# EUROPEAN PATENT SPECIFICATION

(11) **EP 4 054 343 B1**
(45) Date of publication and mention of the grant of the patent: **27.12.2023**
(21) Application number: 19805774.7
(22) Date of filing: 07.11.2019
(51) Int. Cl.: A23C 21/02, A23J 3/34, A23L 33/00, A23L 33/19, A23L 33/18

(54) **EXTENSIVE WHEY PROTEIN HYDROLYSATE WITH TOLEROGENIC PEPTIDES**
UMFANGREICHES MOLKEPROTEINHYDROLYSAT MIT TOLEROGENEN PEPTIDEN
HYDROLYSAT ÉTENDU DE PROTÉINE DE LACTOSÉRUM AVEC DES PEPTIDES TOLÉROGÈNES

(43) Date of publication of application: 14.09.2022
(73) Proprietor: N.V. Nutricia, 2712 HM Zoetermeer (NL)
(72) Inventor: DE OLIVEIRA, Sérgio Bernardo Castro, 3584 CT Utrecht (NL); URBONAITE-ZUKE, Vaida, 3584 CT Utrecht (NL); DEJAX, Clémentine Julie Mélanie, 3584 CT Utrecht (NL); GONSALVES, John Mark Anthony, 3584 CT Utrecht (NL); MANK, Marko, 3584 CT Utrecht (NL); STAHL, Berndt, 3584 CT Utrecht (NL); BALA, Aveenash, 3584 CT Utrecht (NL)
(74) Representative: Nederlandsch Octrooibureau
(86) International application number: PCT/NL2019/050729
(87) International publication number: WO 2021/091369

(56) References cited:
- EP-A1- 0 629 350
- WO-A1-00/42863
- WO-A1-2011/151059
- WO-A1-2013/083140
- WO-A1-2013/083691
- WO-A1-2015/090347
- WO-A1-2016/148572
- JP-A- 2010 105 915
- JOOST W. GOUW ET AL: "Identification of peptides with tolerogenic potential in a hydrolysed whey-based infant formula", CLINICAL & EXPERIMENTAL ALLERGY, vol. 48, no. 10, 1 October 2018 (2018-10-01), pages 1345-1353, XP55713000, UK ISSN: 0954-7894, DOI: 10.1111/cea.13223 cited in the application

## Description

### FIELD

The invention is in the field of formulae comprising hydrolysed cow's milk protein for infants that suffer from or are at risk of developing cow's milk protein allergy.

### BACKGROUND

Dietary proteins are exposed to the immune system in the gastro-intestinal tract. As a default response of the immune system oral tolerance is developed to these innocuous food proteins. The development of oral immune tolerance is highly present in infants, who get exposed to numerous harmless proteins in early life by exposure to dietary proteins. A derail of this natural response to harmless proteins takes place, food allergy will occur. It is generally accepted that the prevalence of food allergy has been increasing in recent decades, particularly in westernised countries. In early life, cow's milk allergy (CMA) is the most dominant food allergy with currently 2-5% of infants suffering. CMA in infancy represents an increasing global health and economic burden, which is caused not only by an increased prevalence over the last decades, but also by an increased persistence, severity and complexity of the condition. Besides acute clinical manifestations, CMA in early life can also have long-lasting effects, including delays in growth and development, as well as increased risk for the development of atopic diseases later in life. Hence, strategies to treat or prevent CMA are of major importance.

The standard dietary management of CMA in infants and children is allergen avoidance through elimination of cow's milk from the diet. A variety of formulas have been developed for the elimination of cow's milk protein. Extensively hydrolysed formula's (eHFs) are recommended for infants with mild CMA, whereas for infants with severe CMA and for infants that either do not tolerate eHFs or in which eHFs fail to resolve CMA symptoms, amino acid-based formulas (AAFs) are recommended. However, such formulae have as a disadvantage that the allergic reaction is merely avoided and not treated. When an infant or child is presented with cow's milk protein, an allergic reaction will occur.

Another approach is to focus on peptides that are able to induce oral immune tolerance to the protein, but are not able to evoke an allergic response. To that end, formulae that contain partially hydrolysed cow's milk protein with peptides which are able to induce oral immune tolerance are marketed. These reduce the risk of an allergic response when intact cow's milk protein is introduced into the diet. However, such formulae typically contain residues of allergens and are not suited for infants already suffering from allergy.

EP 0 629 350 discloses the use of whey protein hydrolysates substantially free of allergens which are said to be capable of inducing cow's milk protein tolerance. A formulae comprising partially hydrolysed whey protein hydrolysate with 3.3 % peptides larger than 5 kDa (Damira^{®}) that was ultrafiltered was able to induce immune tolerance against whole cow's milk protein and alpha lactalbumin when tested with a lymphocyte stimulation test. No effect towards betalactoglobulin was found.

EP 0 827 697 discloses the use of whey, that has been hydrolysed enzymatically for the preparation of compositions that induce oral tolerance to cows' milk in susceptible mammals. The whey has a level of immunological detection of allergenic proteins which is 100 times less than that of unhydrolysed whey.

WO 00/42863 discloses a hypoallergenic composition for the induction of protein tolerance in at risk infants of protein allergy, comprising a non-allergenic extensively hydrolysed protein basis and/or a free amino acid basis, said composition comprising as the active ingredient at least one tolerogenic peptide of the allergenic protein. Examples show that tolerogenic peptides derived from betalactoglobulin have been purified from a hydrolysate or chemically synthesized before adding them to infant formula.

WO 2011/151059 discloses infant nutrition with partially hydrolysed proteins and non-digestible oligosaccharides for use in induction of oral tolerance against native dietary proteins. In particular peptides in the region of the amino acid 13 to 48 of the mature betalactoglobulin molecule were effective in inducing oral tolerance induction. It was shown that the extensively hydrolysed whey protein was not able to induce oral immune tolerance.

WO 2013/083691 discloses synthetic peptides in the region of the amino acid 13 to 48 of the mature betalactoglobulin molecule that are able to treat allergy. Formulae free of allergens to which such peptides are added, are disclosed.

JP 2010-105915 discloses peptides obtained by hydrolyzing betalactoglobulin and being for use in food and drinks.

WO 2015/090347 relates to identified peptides of that are capable of inducing tolerance to cow's milk, especially to betalactoglobulin.

WO 2016/0148572 discloses oral immune tolerance with a specific mixture of synthetic betalactoglobulin peptides and probiotics.

Gouw et al, 2018 Clin Exp Allergy;48:1345-1353, show that a partially whey protein hydrolysate comprises specific tolerogenic peptides in the region of the amino acid 13 to 48 of the mature betalactoglobulin molecule.

WO 2014/14713 discloses an allergen preparation comprising an allergen in an oil-in-water emulsion.

Hence, there exists a need for a nutrition comprising an extensive hydrolysate of whey protein that is allergen-free and contains specific bovine betalactoglobulin peptides and that is produced in an easy and convenient way (without the need of purified or synthetic peptides) that enhance the oral immune tolerance for subjects suffering from food allergy with improved effects on oral immune tolerance induction against food proteins, in particular cow's milk protein.

### SUMMARY OF THE INVENTION

It is customary in the art to subject whey protein hydrolysate to an ultrafiltration step to reduce the amount of allergenic protein, especially betalactoglobulin. As the size of the protein is larger than the size of the peptides, a pore size is selected that is impermeable to the large protein but allows the passage of the smaller peptides, thus expecting the tolerogenic peptides to permeate. However, applying such traditional technology the inventors found that tolerogenic betalactoglobulin peptides in the region of amino acid 13-48 of the mature betalactoglobulin molecule were *not* present in conventional ultrafiltered whey protein hydrolysate. While it was found that these peptides were in fact generated in the first step where the protein was degraded by proteolytic enzymes, none was present in the permeate after the membrane filtration step, and instead remained in the retentate as demonstrated in example 1. This retention cannot be explained by the size of the peptides, which was well below the cut-off value of the ultrafiltration membrane.

Therefore, the inventors developed an improved method for producing ultrafiltered whey protein hydrolysate. The inventors surprisingly found that by adding food-grade emulsifiers (example 2) or chaotropic agents (example 3) before, during or after whey protein hydrolysis but *before* the membrane filtration step the tolerogenic betalactoglobulin peptides were able to pass the membrane and appeared in the permeate. This results in hydrolyzed whey protein that is enriched in tolerogenic betalactoglobulin peptides, without the present of residues of larger allergenic peptides and proteins, such as immunogenic betalactoglobulin.

In a first aspect, the invention pertains to a process for preparing an ultrafiltered whey protein hydrolysate, said process comprising:
a) subjecting whey protein **A** to hydrolysis **1** with one or more proteolytic enzymes thereby obtaining a whey protein hydrolysate **B**, and
b) subjecting the whey protein hydrolysate **B** to membrane ultrafiltration **2** thereby obtaining a permeate **C1** and a retentate **C2,**
wherein:
(i) an emulsifier **E** is added to the whey protein **A** prior to or during hydrolysis **1** or wherein an emulsifier and/or a chaotropic agent **E** is added to the whey protein hydrolysate **B** prior to the membrane ultrafiltration **2,**
wherein the ultrafiltered whey protein hydrolysate is in permeate **C1,**
and/or wherein:
(ii) after step b)
c) an emulsifier **E** and/or a chaotropic agent **E** is added to retentate **C2** thereby obtaining a retentate **C3,**
d) subjecting the retentate **C3** to membrane ultrafiltration **3** thereby obtaining a permeate **D1** and a retentate **D2,** and
e) adding at least part of the permeate **D1** to the permeate **C1** thereby obtaining permeate **D3,** wherein the ultrafiltered whey protein hydrolysate is in permeate **D3.**

The whey protein **A** provided in step a) may originate from one or more whey protein sources.

Not bound by theory the inventors believe the tolerogenic fragments cluster in the absence of an emulsifier and/or chaotropic agent **E.** The size of the clusters is such that they do not permeate the ultrafiltration membrane. By adding an emulsifier and/or chaotropic agent **E** it is believed cluster formation is prevented or reversed; enabling permeation upon ultrafiltration. As chaotropic agents may affect enzymes (which are proteins themselves), preferably the chaotropic agent is added only after hydrolysis in step (i), and/or in step (ii).

In a second aspect, the invention pertains an ultrafiltered whey protein hydrolysate obtainable by the process described above and herein. More particularly, the invention relates to an ultrafiltered whey protein hydrolysate having:
- less than 6 microgram allergenic beta-lactoglobulin per gram protein; and/or less than 3%, preferably less than 1% of peptides with a size of 5kDa based on total protein, and
- at least one betalactoglobulin peptide comprising an amino acid sequence according to any one of SEQ ID No. 2 - 30, preferably according to any one of SEQ ID No. 31 - 57, more preferably according to any one of SEQ ID No. 58-70.

In one embodiment, the ultrafiltered whey protein hydrolysate is an extensively hydrolyzed, ultrafiltered whey protein hydrolysate having:
- less than 0.8 microgram, preferably less than 0.2 microgram allergenic beta-lactoglobulin per gram protein; and/or less than 3%, preferably less than 1% of peptides with a size of 5kDa based on total protein, and
- at least one betalactoglobulin peptide comprising an amino acid sequence according to any one of SEQ ID No. 2 - 70, preferably according to any one of SEQ ID No. 2 - 30, more preferably according to any one of SEQ ID No. 31-57, more preferably according to any one of SEQ ID No. 58-70. Further preferences are found in the detailed description.

Here above, the amount of peptides based on total protein may be determined using size exclusion high pressure liquid chromatography as known in the art. Saint-Sauveur et al. "Immunomodulating properties of a whey protein isolate, its enzymatic digest and peptide fractions" Int. Dairy Journal (2008) vol. 18(3) pages 260-270 describes an example thereof. In short, the total surface area of the chromatograms is integrated and separated into mass ranges expressed as percentage of the total surface area. The mass ranges are calibrated using peptides/proteins with a known molecular mass.

The ultrafiltered hydrolysate may preferably comprise at least 25 mg, more preferably at least 50 mg chaotropic agent per gram protein; and/or at least 5 mg emulsifier per gram protein, which is indicative of the process of the invention.

Advantageously, the ultrafiltered whey protein contains only reduced levels of allergenic protein, yet contains therapeutically effective amounts of tolerogenic peptides.

In a third aspect, the invention pertains to the use of the improved ultrafiltered whey protein hydrolysate in the manufacture of infant or young child formulae for treating infants or young children that suffer from bovine, ovine and/or caprine milk protein allergy, in order to treat allergy and induce oral immune tolerance against the bovine, ovine and/or caprine milk protein. Moreover as the tolerogenic fragments also have a proven effect in preventing allergy, the improved ultrafiltered whey protein hydrolysate can advantageously be used for the preparation of infant or young child formulae, in order to prevent bovine, ovine and/or caprine milk protein allergy, and in order to induce oral immune tolerance against bovine, ovine and/or caprine milk protein, especially if the infant or young child is at risk of bovine, ovine and/or caprine milk protein allergy. The allergy is preferably cow's milk protein allergy.

### LIST OF FIGURES

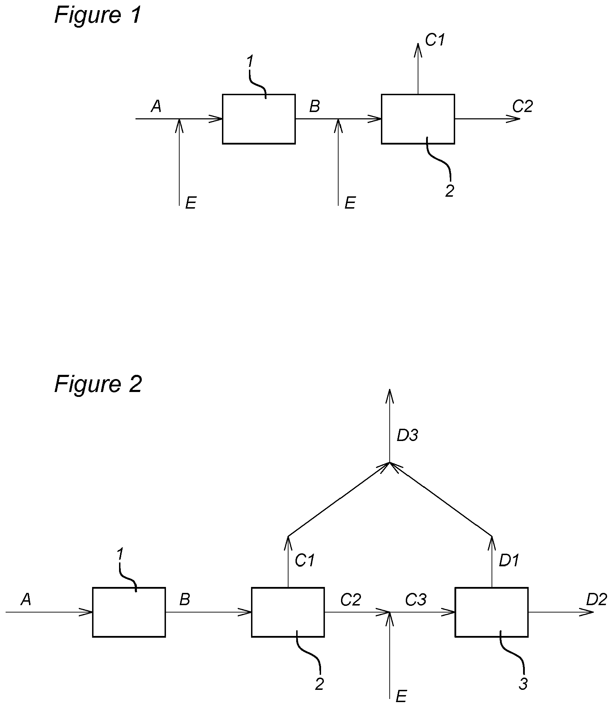

Figure 1 provides a schematic overview of the manufacturing process according to one embodiment of the invention. A whey protein **A** is provided that is first hydrolyzed in step **1,** wherein whey protein hydrolysate **B** is obtained. Subsequently whey protein hydrolysate **B** is subjected to ultrafiltration **2** yielding permeate **C1** and retentate **C2.** The ultrafiltered whey protein hydrolysate is in permeate **C1.** The chaotropic agent **E** and/or emulsifier **E** is added before, during and/or after step **1** but before step **2.**

Figure 2 provides a schematic overview of the manufacturing process according to another preferred embodiment of the invention. A whey protein **A** is provided that is first hydrolyzed in step **1,** wherein whey protein hydrolysate **B** is obtained. Subsequently whey protein hydrolysate **B** is subjected to ultrafiltration **2** yielding permeate **C1** and retentate **C2.** The chaotropic agent and/or emulsifier **E** is subsequently added to retentate **C2** to obtain retentate **C3.** Retentate **C3** is subjected to ultrafiltration **3** yielding permeate **D1** and retentate **D2.** At least a part of permeate **D1** is combined with retentate **C1** to obtain permeate **D3** wherein the ultrafiltered whey protein hydrolysate is in permeate **D3.** Additionally the chaotropic agent **E** and/or emulsifier **E** may be added before, during and/or after step **1** but before step **2.**

### DETAILED DESCRIPTION

The invention thus concerns a process for preparing an ultrafiltered whey protein hydrolysate, said process comprising:
a) Subjecting whey protein **A** to hydrolysis **1** with one or more proteolytic enzymes thereby obtaining a whey protein hydrolysate **B**, and
b) subjecting the whey protein hydrolysate **B** to membrane ultrafiltration **2** thereby obtaining a permeate **C1** and a retentate **C2,**
wherein
(i) an emulsifier **E** is added to the whey protein **A** prior to or during hydrolysis **1** or wherein an emulsifier and/or a chaotropic agent **E** is added to the whey protein hydrolysate **B** prior to the membrane ultrafiltration **2,**
wherein the ultrafiltered whey protein hydrolysate is in permeate **C1,**
and/or wherein
(ii) after step b)
c) an emulsifier and/or a chaotropic agent **E** is added to retentate **C2** thereby obtaining a retentate **C3,**
d) subjecting the retentate **C3** to membrane ultrafiltration **3** thereby obtaining a permeate **D1** and a retentate **D2,** and
e) adding at least part of the permeate **D1** to the permeate **C1** thereby obtaining permeate **D3,**
wherein the ultrafiltered whey protein hydrolysate is in permeate **D3.**

The invention also concerns an ultrafiltered whey protein hydrolysate obtainable by this process.

Furthermore the invention concerns an ultrafiltered whey protein hydrolysate, wherein the ultrafiltered whey protein hydrolysate comprises peptides with the following size distribution:
60 - 90 % with a size < 1 kDa,
10 - 40 % of peptides with a size of 1 to < 5 kDa,
0 - 0.5 % of peptides with a size of 5 to <10 kDa, and
0 - 0.2 % of peptides with a size > 10 kDa,
based on the total protein of the hydrolysate, and
wherein the ultrafiltered whey protein hydrolysate comprises:
∘ less than 6 microgram allergenic beta-lactoglobulin per gram protein; and/or less than 3%, preferably less than 1% of peptides with a size of 5kDa based on total protein, and
∘ at least one, preferably at least two, more preferably at least three of betalactoglobulin peptide(s) comprising an amino acid sequence according to any one of SEQ ID No. 66 -70.

The invention also concerns a whey protein hydrolysate composition comprising ultrafiltered whey protein hydrolysate, in a concentration of at least 0.8 gram of ultrafiltered whey protein hydrolysate per gram protein, wherein the ultrafiltered whey protein hydrolysate comprises:
- less than 6 microgram allergenic beta-lactoglobulin per gram protein; and/or less than 3%, preferably less than 1% of peptides with a size of 5kDa based on total protein, and
- at least one betalactoglobulin peptide comprising an amino acid sequence according to any one of SEQ ID No. 2 - 70, preferably according to any one of SEQ ID No. 31-70; and
- preferably at least 50 mg urea per gram protein and/or at least 5 mg emulsifier per gram protein.

Furthermore the invention concerns a nutritional composition comprising the ultrafiltered whey protein hydrolysate or the whey protein hydrolysate composition, wherein said composition is an infant formula, a follow-on formula or a young child formula, preferably an infant formula or a follow-on formula.

The invention also concerns the use of the nutritional composition in the manufacture of a product (i) for providing nutrition to; and/or
(ii) for treating/preventing allergy in; and/or
(iii) for inducing oral immune tolerance against an allergen, preferably a food allergen in subjects suffering from allergy, preferably suffering from food allergy, preferably bovine, ovine and/or caprine milk protein, preferably allergy allergy selected from the group consisting of cow's milk protein allergy, goat's milk protein allergy and sheep's milk protein allergy,
wherein the subjects are preferably children up to 6 years of age, more preferably infants between 0 and 12 months of age.

Also the invention concerns the use of the ultrafiltered whey protein hydrolysate or the whey protein hydrolysate composition in the manufacture of a nutritional composition, wherein said composition is preferably an infant formula, a follow-on formula or a young child formula, preferably an infant formula or a follow-on formula.

### Protein levels

In the context of the present invention, protein is defined as the sum of protein, peptides and free amino acids. Protein according to the present invention can also be referred to as protein equivalent. Where numbers are presented based on protein, it thus means that it is based on the sum of all proteinaceous materials including protein, peptides and free amino acids. Amino acids are the 20 different natural amino acids that can be part of eukaryotic protein and peptides. In the context of the invention, amino acids are considered free amino acids, distinct from peptides. The level of total protein is derived from the commonly known Kjeldahl nitrogen analysis and multiplication by a factor of 6.25. This gives a level of total protein, sometimes also referred to as crude protein level. Hence total protein is the sum of protein equivalent as defined herein plus non-protein nitrogen (NPN). Methods to determine protein and peptides and free amino acids are known in the art. For example a method wherein first protein an peptides are precipitated followed by common protein determination and free amino acids can be determined in the non-precipitated fraction by HPLC with pre-column derivatization with fluorenylmethyl-chloroformate and UV and fluorescence detection is suitable.

### Whey protein (A)

In the context of the current invention whey protein is derived from milk from mammals. The whey protein **A** is preferably derived from milk from a species of the genus *Bos, Bison, Bubalus* or *Capra,* more preferably from genus Bos, preferably from cow's milk. The whey protein **A** may be derived from one or more different milk sources. In other words, the whey protein is preferably *Bos, Bison, Bubalus* or *Capra* whey protein, or combinations thereof, more preferably Bos whey protein, preferably cow's milk whey protein. Consequently the whey protein hydrolysate **B** or the ultrafiltered whey protein hydrolysate is preferably derived from milk from a species of the genus *Bos, Bison, Bubalus* or *Capra,* more preferably from genus Bos, preferably from cow's milk. In other words, the whey protein hydrolysate **B** or the ultrafiltered whey protein hydrolysate is preferably *Bos, Bison, Bubalus* or *Capra* (ultrafiltered) whey protein hydrolysate, more preferably Bos (ultrafiltered) whey protein hydrolysate, preferably cow's milk (ultrafiltered) whey protein hydrolysate.

Preferably, the whey protein **A** is one or more of selected from the group consisting of acid whey protein, sweet whey protein, whey protein concentrate (WPC), whey protein isolate (WPI) or demineralized whey powder. These whey types are commercially available. A suitable source is a mixture of acid whey protein and demineralised sweet whey protein. Sweet whey is the by-product of rennet-coagulated cheese and comprises caseinoglycomacropeptide (CGMP), and acid whey (also called sour whey) is the by-product of acid-coagulated cheese, and does not contain CGMP. Suitable sources for the whey protein are demineralised whey (Deminal, Friesland Campina, the Netherlands) and/or whey protein concentrate (WPC80, Friesland Campina, the Netherlands). The whey protein to be hydrolyzed preferably comprises acid whey, more preferably at least 50 wt%, more preferably at least 70 wt% acid whey, based on total whey protein. Acid whey has an improved amino acid profile compared to sweet whey protein. The source of whey protein is not critical to the process of the invention.

Whey protein **A** comprises a considerable amount of betalactoglobulin (BLG). The amino acid sequence of BLG in species of *Bos* and *Bison* are similar. In species of *Capra* and *Bubalus* the amino acid at position 1 is different (an isoleucine instead of a leucine). The at least one betalactoglobulin peptide comprising or having an amino acid sequence according to any one of SEQ ID No. 2 - 70, preferably according to any one of SEQ ID No. 31-70, more preferably according to any one of SEQ ID no. 2-24, 31-51 and 66-70, even more preferably according to any one of SEQ ID no. 31-51 and 66-70, particularly preferably according to any one of SEQ ID no. 2-17, 31-44, 66 and 68-70, even more preferably according to any one of SEQ ID no. 31-44, 66 and 68-70, most preferably according to any of SEQ ID no. 66 and 68-70 have the same tolerogenic properties regardless the source of whey protein.

### Hydrolysis (1)

Hydrolysis concerns protein hydrolysis and is performed with one or more proteolytic enzymes. The proteolytic enzymes used for the hydrolysis is from animal or vegetable origins (e.g. pepsin, chymotrypsin, trypsin, intestinal mucosa extract, pancreatic extracts, chymosin, papain, bromelain, ficin), bacterial or fungi origins (e.g. serine and metallo-5 proteases from *Bacillus subtilis, Bacillus licheniformis, Bacillus amyloliquefaciens, Aspergillus orysae, Aspergillus wentïi* and acidic proteases from *Aspergillus orizae, Aspergillus wentii, Mucor miehei, Mucor pusillus, Endothia parasitica)* or a combination of these. By way of example, the proteolytic enzyme can be alcalase, acidic proteases from *Aspergillus oryzae* or trypsin

During hydrolysis, the concentration of the protein in solution or in suspension is preferably in the range of 2-20% by weight (wt%),and may be pasteurized before introducing proteases. The ratio enzyme/protein preferably is between 0.1-10% weight/weight, more preferably between 0.25 to 4% weight/weight. The skilled person has the skills and knowledge to carry out conventional enzymatic hydrolysis. Hydrolysis preferably is conducted at a temperature of about 35 °C to 65 °C. Hydrolysis is preferably conducted between 30 minutes and 10 hours, more preferably between 30 min to 4 hours. The pH during hydrolysis is between 2.5 and 11, preferably between 5 and 9, more preferably between 6 and 8.5. If desired the pH of the solution can be adjusted and regulated with citric acid, food grade HCl or NaOH, NH4OH, KOH, Ca(OH)2 and/or Mg(OH)2, for instance at a concentration of 2N pure or in blend.

The enzymatic process of hydrolysis may be stopped by fast cooling. Preferably the whey protein hydrolysate obtained after hydrolysis (or obtained in step a) is cooled before any subsequent process step, such a membrane ultrafiltration. Preferably the whey protein hydrolysate is cooled below 30°C, more preferably below 20°C, most preferably below 10°C. Alternatively or additionally, the protein hydrolysate preferably is subjected to a heat treatment of about 0.01 to 10 min at a temperature of about 70 to 130°C to inactivate residual enzymes, i. e. proteases. Cooling may precede and/or succeed the heat treatment. The whey protein hydrolysate **B** thus obtained may be clarified by centrifugation or filtration to remove insoluble proteins, and a clear solution is recovered.

The recovered (clear) hydrolysate solution can, if desired, be concentrated by evaporation to a dry solid content of for instance 10-50% (weight/weight) for a subsequent treatment or spray dried.

### Membrane ultrafiltration

The method of the current invention also involves membrane ultrafiltration (UF), after hydrolysis 1. That is, the whey protein hydrolysate obtained here above is subsequently subjected to UF. All mentioned with regard to UF here below applies to membrane ultrafiltration 2 and 3 as detailed in figures 1 and 2.

The membrane ultrafiltration (or ultrafiltration) 2 and/or 3 can be carried out with any UF membrane known in the art, including flat sheet membranes, ceramic membranes, tubular and organic spiral wound membranes. Preferably the UF membrane is a spiral wound membrane. The UF membrane has a molecular weight cut-off of that enables intact proteins, preferably allergenic betalactoglobulin, to remain in the retentate, and allow peptides to permeate through the membrane. The skilled person is well capable of determining the appropriate cut-off. The membrane ultrafiltration 2 and/or 3 is preferably carried out with a membrane having a molecular weight cut-off of at most 15 kDa, more preferably 5 - 11 kDa, more preferably at least 9 kDa. The membrane ultrafiltration 2 and/or 3 are preferably carried out with a membrane having a pore size with a cut-off of 5 - 15 kDa, preferably 5-11 kDa, more preferably 9-11 kDa.

The membrane ultrafiltration 2 and/or 3 is preferably performed below 40 °C, more preferably between 3 °C and 30 °C, even more preferably between 5 °C and 20 °C.

The membrane ultrafiltration 2 and/or 3 is preferably carried out with a volume concentration factor (VCF) in the range of 5 - 50, more preferably 10 - 25, which has been found to provide the most optimal results in terms of the composition of the UF permeate. In the context of the invention, the term "volume concentration factor" or "VCF" is the factor at which a liquid composition is concentrated upon filtration, i.e. the total volume of the incoming stream prior to filtration divided by the total volume of the retentate after filtration, irrespective of the total solid content. Thus, when 100 kg of a liquid composition is fractionated over an ultrafiltration membrane into a permeate of 93 kg and a retentate of 7 kg, this UF process operates with a VCF of 100/7 of about 15.

Using such processing parameters, the UF permeate stream **C1** typically comprises at least 80% (w/w), preferably at least 80% (w/w) more preferably 90% (w/w) of the whey protein **A.** The ultrafiltration 3 will mainly deliver tolerogenic peptides from retentate **C3.**

### The ultrafiltered whey protein hydrolysate

In the context of the current invention - and in accordance with the art - the term `peptide size' and 'peptide weight' may be used interchangeably. Both peptide size and peptide weight are expressed in Dalton (Da) or kiloDalton (kDa).

Other weights are expressed in kilogram (kg), gram (g), milligram (mg) and microgram (mcg or µg); the abbreviation of these units are between brackets.

The hydrolysis 1 will mainly deliver peptides. Subsequently ultrafiltration 2 will further reduce the amount of any intact protein in the permeate. The peptides mix may be characterized by its size distribution or degree of hydrolysis. Consequently the ultrafiltered whey protein hydrolysate according to the present invention may be characterized by a peptide size distribution or degree of hydrolysis. The size distribution does not need to encompass a full distribution. The presence of specific amounts of peptides of one or more specific size ranges may be sufficient for characterization.

In the art sometimes whey protein hydrolysate is further characterized in partial whey protein hydrolysate or partially hydrolyzed whey protein or `pHP', and extensively whey protein hydrolysate or extensively hydrolyzed whey protein or `eHP'. If such protein is taken up in a formula, common naming is pHF for formula comprising partially hydrolyzed whey protein and eHF for formula comprising extensively hydrolyzed whey protein. The skilled person understands the overall peptide size in eHP will be smaller compared to pHP. In the context of the current invention eHP is characterized as being a whey protein hydrolysate with less than 1% of peptides with a size of 5kDa based on total protein and pHP less than 3% of peptides with a size of 5kDa based on total protein.

Preferably the ultrafiltered whey protein hydrolysate **B** is extensively hydrolyzed whey protein. eHP can be obtained by hydrolysis of whey proteins to a high degree of hydrolysis (substantial hydrolysis) or by removing the larger peptides of hydrolyzed whey protein by ultrafiltration. Disadvantage of substantial hydrolysis is that on one side in the context of allergy still significant amounts of intact allergic protein such as allergic betalactoglobulin may be present and that on the other side the protein is hydrolyzed to such extend that no or only a small amount of tolerogenic peptides will be present as for peptides to be tolerogenic they need a minimum size. Although ultrafiltration of a whey protein hydrolysate resolves the issue of remnant intact allergic protein, an additional problem appears, as the tolerogenic peptides do not permeate the ultrafiltration membrane. Therefore the beneficial tolerogenic properties will be lost.

The ultrafiltered whey protein hydrolysate or the whey protein hydrolysate preferably has a degree of hydrolysis (of the protein) between 5 and 50 %, more preferably between 10 and 30 %, even more preferably between 15 and 25 %. The degree of hydrolysis is defined as the percentage of peptide bonds which have been broken down by enzymatic hydrolysis, with 100 % being the total potential peptide bonds present. A too high degree of hydrolysis will result in an undesirable low level or even absence of tolerogenic peptides, whilst a too low degree of hydrolysis will deliver an undesirable high level of intact allergic protein. When not filtered out the intact protein may induce an allergic reaction. At the same time, filtration would result in unattractively low yields in case of an ultrafiltered whey protein hydrolysate with too low degree of hydrolysis (as too much protein remains in the retentate). Proteins with the abovementioned degree of hydrolysis provide sufficient peptides with a chain length of 2 to 30.

The peptide size and molecular weight distribution can be determined by routine methods known to the skilled person such as HPLC or size exclusion chromatography (SEC), in particular high performance size exclusion chromatography. Saint-Sauveur et al. "Immunomodulating properties of a whey protein isolate, its enzymatic digest and peptide fractions" Int. Dairy Journal (2008) vol. 18(3) pages 260-270 describes an example thereof. In short, the total surface area of the chromatograms is integrated and separated into mass ranges expressed as percentage of the total surface area. The mass ranges are calibrated using peptides/proteins with a known molecular mass. The protein hydrolysate comprising said peptides is preferably characterised in that it comprises between 55 and 85 % peptides with a molecular weight below 1000 Da, between 15-45 % peptides with a molecular weight between 1000 and below 5000 Da and between 0 and 1 % of peptides or proteins with a molecular weight of 5000 Da and above, all based on total protein of the hydrolysate.

In a preferred embodiment the ultrafiltered whey protein hydrolysate is characterized in that the hydrolysate comprises peptides with the following size distribution:
60 - 90 % with a size < 1 kDa,
10 - 40 % of peptides with a size of 1 to < 5 kDa,
0 - 0.5 % of peptides with a size of 5 to <10 kDa, and
0 - 0.2 % of peptides with a size > 10 kDa,
based on the total protein of the hydrolysate.

In a preferred embodiment of the present invention the ultrafiltered whey protein hydrolysate is characterized in that it comprises less than 100mg per gram protein, preferably less than 50mg per gram protein, more preferably less than 10mg per gram protein of peptides with a size above 5 kDa. Preferably the ultrafiltered whey protein hydrolysate comprises less than 30 mg per gram protein, preferably less than 10 mg per gram protein, most preferably less than 5 mg per gram protein of peptides with a size above 10 kDa.

Preferably, the ultrafiltered whey protein hydrolysate comprises at least 300 mg of peptides with a size of 1 kDa or below per gram protein, even more preferably at least 500 mg per gram protein. Preferably the ultrafiltered whey protein hydrolysate comprises at least 900 mg per gram protein of peptides with a size of 5 kDa or below, even more preferably at least 950 mg per gram protein, most preferably at least 990 mg per gram protein.

The ultrafiltered whey protein hydrolysate comprises no synthetic peptides, more preferably no synthetic tolerogenic peptides. The ultrafiltered whey protein hydrolysate does not comprise purified tolerogenic peptides. Purification of tolerogenic peptides and purified tolerogenic peptides are known in the art; a purification method and purified tolerogenic peptides is for example described in WO 00/42863.

### Tolerogenic peptides

The ultrafiltered whey protein hydrolysate comprises tolerogenic peptides. Tolerogenic peptides are peptides that induce immune tolerance towards undesirable activation of the immune response towards the protein of which the peptide is a fragment of. Betalactoglobulin is a known allergen. The amino acid 13-48 region of betalactoglobulin is known for its tolerogenic potential (Gouw et al., 2018, Clin. Exp. Allergy 48:1345-1253). In this study of Gouw et al. peptides having a sequence of in the 13-48 region of betalactoglobulin were found tolerogenic. WO 00/42863 A1 discloses betalactoglobulin peptides having a sequence of 8 overlapping amino acids with betalactoglobulin to be tolerogenic. WO 2011/151059 A1 discloses betalactoglobulin peptides having a sequence of 17 amino acids overlapping with the 13-48 region of betalactoglobulin to be tolerogenic. JP 2010/105915 A1 discloses betalactoglobulin peptides having a sequence of 19 amino acids having 8 AA overlap with the 13-48 region of betalactoglobulin to be tolerogenic. WO 2015/090347 A1 discloses betalactoglobulin peptides having a sequence of 6-9 amino acids having at least 7 AA overlap with the 13-48 region of betalactoglobulin to be tolerogenic. WO 2016/148572 A1 discloses betalactoglobulin peptides having at least 8 AA overlap with the 13-48 region of betalactoglobulin to be tolerogenic. In general the tolerogenic effect is related to that tolerogenic peptides are T-cell epitopes and bind to T-cells, for example on HLA-DR alleles. In this way oral tolerance is induced. For the tolerogenic peptides to efficiently act as T-cell epitope a minimum size of 8 AA is generally assumed.

Determination of amino acid sequence of betalactoglobulin peptides including tolerogenic peptides in the ultrafiltered whey protein hydrolysate can for example be determined according to Gouw et al., 2018, Clin. Exp. Allergy 48:1345-1253, optionally modified that instead of nanoLC-ESI MS2, micro LC-MS ESI MS2 is used.

In a preferred embodiment the ultrafiltered whey protein hydrolysate comprises at least one betalactoglobulin peptide comprising an amino acid sequence with a length of 8 amino acids according to any one SEQ ID no. 2-30, more preferably the ultrafiltered whey protein hydrolysate comprises at least one betalactoglobulin peptide comprising an amino acid sequence with a length of 10 amino acids according to any one SEQ ID no. 31-57. Preferably the ultrafiltered whey protein hydrolysate comprises at least two, even more preferably at least three peptides comprising an amino acid sequence according to any one of SEQ ID no. 2-30. More preferably the ultrafiltered whey protein hydrolysate comprises at least two, even more preferably at least three peptides comprising an amino acid sequence according to any one of SEQ ID no. 31-57.

In a further preferred embodiment, the ultrafiltered whey protein hydrolysate comprises at least one betalactoglobulin peptide comprising an amino acid sequence according to any one of SEQ ID No. 58 - 70, more preferably the ultrafiltered whey protein hydrolysate comprises at least one betalactoglobulin peptide comprising an amino acid sequence according to any one of SEQ ID No. 66 - 70, most preferably the ultrafiltered whey protein hydrolysate comprises at least one betalactoglobulin peptide comprising an amino acid sequence according to any one of SEQ ID No. 66 and 68 - 70. In a most preferred embodiment, the ultrafiltered whey protein hydrolysate comprises at least two, preferably at least three, more preferably at least four, even more preferably at least five of the betalactoglobulin peptide comprising an amino acid sequence according to any one of SEQ ID No 58-70.

In a further preferred embodiment, the ultrafiltered whey protein hydrolysate comprises at least one betalactoglobulin peptide comprising an amino acid sequence according to any one of SEQ ID No. 2-24, 31-51 and 66-70, even more preferably according to any one of SEQ ID no. 31-51 and 66-70, particularly preferably according to any one of SEQ ID no. 2-17, 31-44, 66 and 68-70, even more preferably according to any one of SEQ ID no. 31-44, 66 and 68-70.

More preferably, the ultrafiltered whey protein hydrolysate comprises at least two, even more preferably at least three peptides comprising an amino acid sequence according to any one of SEQ ID No. 2 - 70, preferably according to any one of SEQ ID No. 31-70. More preferably, the ultrafiltered whey protein hydrolysate comprises at least two, even more preferably at least three peptides comprising an amino acid sequence according to any one of SEQ ID no. 2-24, 31-51 and 66-70. Even more preferably, the ultrafiltered whey protein hydrolysate comprises at least two, even more preferably at least three peptides comprising an amino acid sequence according to any one of SEQ ID no. 31-51 and 66-70. More preferably, the ultrafiltered whey protein hydrolysate comprises at least two, even more preferably at least three peptides comprising an amino acid sequence according to any one of SEQ ID no. 2-17, 31-44, 66 and 68-70. More preferably, the ultrafiltered whey protein hydrolysate comprises at least two, even more preferably at least three peptides comprising an amino acid sequence according to any one of SEQ ID no. 31-44, 66 and 68-70, most preferably according to any of SEQ ID no. 66 and 68-70.

In a preferred embodiment, the ultrafiltered whey protein hydrolysate comprises at least one betalactoglobulin peptide comprising an amino acid sequence according to any one of SEQ ID No. 2 - 57 and 66-70, preferably according to any one of SEQ ID No. 31-57 and 66-70, even more preferably according to any one of SEQ ID no. 31-51 and 66-70. More preferably, the ultrafiltered whey protein hydrolysate comprises at least two, even more preferably at least three peptides comprising an amino acid sequence according to any one of SEQ ID No. 2 - 57 and 66-70. More preferably, the ultrafiltered whey protein hydrolysate comprises at least two, even more preferably at least three peptides comprising an amino acid sequence according to any one of SEQ ID No. 31-57 and 66-70. More preferably, the ultrafiltered whey protein hydrolysate comprises at least two, even more preferably at least three peptides comprising an amino acid sequence according to any one of SEQ ID no. 31-51 and 66-70.

The at least one specific betalactoglobulin peptide is preferably a natural peptide. A natural peptide is defined as being obtained from hydrolysis of naturally occurring protein. A synthetic peptide is defined as being chemically synthesized.

Throughout the specification and claims, the amino acid sequences of the BLG-peptides with SEQ ID NO: 1 - 70 are identified here below:

| | | |
|---|---|---|
| SEQ ID NO: 1 | AA 1-48 | XIVTQTMKGLDIQKVAGTWYSLAMAASDISLLDAQSAPLRVYVEELKP |
| SEQ ID NO: 2 | AA 13-20 | QKVAGTWY |
| SEQ ID NO: 3 | AA 14-21 | KVAGTWYS |
| SEQ ID NO: 4 | AA 15-22 | VAGTWYSL |
| SEQ ID NO: 5 | AA 16-23 | AGTWYSLA |
| SEQ ID NO: 6 | AA 17-24 | GTWYSLAM |
| SEQ ID NO: 7 | AA 18-25 | TWYSLAMA |
| SEQ ID NO: 8 | AA 19-26 | WYSLAMAA |
| SEQ ID NO: 9 | AA 20-27 | YSLAMAAS |
| SEQ ID NO: 10 | AA 21-28 | SLAMAASD |
| SEQ ID NO: 11 | AA 22-29 | LAMAASDI |
| SEQ ID NO: 12 | AA 23-30 | AMAASDIS |
| SEQ ID NO: 13 | AA 24-31 | MAASDISL |
| SEQ ID NO: 14 | AA 25-32 | AASDISLL |
| SEQ ID NO: 15 | AA 26-33 | ASDISLLD |
| SEQ ID NO: 16 | AA 27-34 | SDISLLDA |
| SEQ ID NO: 17 | AA 28-35 | DISLLDAQ |
| SEQ ID NO: 18 | AA 29-36 | ISLLDAQS |
| SEQ ID NO: 19 | AA 30-37 | SLLDAQSA |
| SEQ ID NO: 20 | AA 31-38 | LLDAQSAP |
| SEQ ID NO: 21 | AA 32-39 | LDAQSAPL |
| SEQ ID NO: 22 | AA 33-40 | DAQSAPLR |
| SEQ ID NO: 23 | AA 34-41 | AQSAPLRV |
| SEQ ID NO: 24 | AA 35-42 | QSAPLRVY |
| SEQ ID NO: 25 | AA 36-43 | SAPLRVYV |
| SEQ ID NO: 26 | AA 37-44 | APLRVYVE |
| SEQ ID NO: 27 | AA 38-45 | PLRVYVEE |
| SEQ ID NO: 28 | AA 39-46 | LRVYVEEL |
| SEQ ID NO: 29 | AA 40-47 | RVYVEELK |
| SEQ ID NO: 30 | AA 41-48 | VYVEELKP |

| | | |
|---|---|---|
| SEQ ID NO: 31 | AA 13-22 | QKVAGTWYSL |
| SEQ ID NO: 32 | AA 14-23 | KVAGTWYSLA |
| SEQ ID NO: 33 | AA 15-24 | VAGTWYSLAM |
| SEQ ID NO: 34 | AA 16-25 | AGTWYSLAMA |
| SEQ ID NO: 35 | AA 17-26 | GTWYSLAMAA |
| SEQ ID NO: 36 | AA 18-27 | TWYSLAMAAS |
| SEQ ID NO: 37 | AA 19-28 | WYSLAMAASD |
| SEQ ID NO: 38 | AA 20-29 | YSLAMAASDI |
| SEQ ID NO: 39 | AA 21-30 | SLAMAASDIS |
| SEQ ID NO: 40 | AA 22-31 | LAMAASDISL |
| SEQ ID NO: 41 | AA 23-32 | AMAASDISLL |
| SEQ ID NO: 42 | AA 24-33 | MAASDISLLD |
| SEQ ID NO: 43 | AA 25-34 | AASDISLLDA |
| SEQ ID NO: 44 | AA 26-35 | ASDISLLDAQ |
| SEQ ID NO: 45 | AA 27-36 | SDISLLDAQS |
| SEQ ID NO: 46 | AA 28-37 | DISLLDAQSA |
| SEQ ID NO: 47 | AA 29-38 | ISLLDAQSAP |
| SEQ ID NO: 48 | AA 30-39 | SLLDAQSAPL |
| SEQ ID NO: 49 | AA 31-40 | LLDAQSAPLR |
| SEQ ID NO: 50 | AA 32-41 | LDAQSAPLRV |
| SEQ ID NO: 51 | AA 33-42 | DAQSAPLRVY |
| SEQ ID NO: 52 | AA 34-43 | AQSAPLRVYV |
| SEQ ID NO: 53 | AA 35-44 | QSAPLRVYVE |
| SEQ ID NO: 54 | AA 36-45 | SAPLRVYVEE |
| SEQ ID NO: 55 | AA 37-46 | APLRVYVEEL |
| SEQ ID NO: 56 | AA 38-47 | PLRVYVEELK |
| SEQ ID NO: 57 | AA 39-48 | LRVYVEELKP |

| | | |
|---|---|---|
| SEQ ID NO: 58 | AA 1-22 | XIVTQTMKGLDIQKVAGTWYSL |
| SEQ ID NO: 59 | AA 1-25 | XIVTQTMKGLDIQKVAGTWYSLAMA |

| | | |
|---|---|---|
| SEQ ID NO: 60 | AA 1-26 | XIVTQTMKGLDIQKVAGTWYSLAMAA |
| SEQ ID NO: 61 | AA 1-30 | XIVTQTMKGLDIQKVAGTWYSLAMAASDIS |
| SEQ ID NO: 62 | AA 1-35 | XIVTQTMKGLDIQKVAGTWYSLAMAASDISLLDAQ |
| SEQ ID NO: 63 | AA 1-39 | XIVTQTMKGLDIQKVAGTWYSLAMAASDISLLDAQSAPL |
| SEQ ID NO: 64 | AA 1-42 | XIVTQTMKGLDIQKVAGTWYSLAMAASDISLLDAQSAPLRVY |
| SEQ ID NO: 65 | AA 5-35 | QTMKGLDIQKVAGTWYSLAMAASDISLLDAQ |

| | | |
|---|---|---|
| SEQ ID NO: 66 | AA 1-27 | XIVTQTMKGLDIQKVAGTWYSLAMAAS |
| SEQ ID NO: 67 | AA 11-42 | DIQKVAGTWYSLAMAASDISLLDAQSAPLRVY |
| SEQ ID NO: 68 | AA 6-35 | TMKGLDIQKVAGTWYSLAMAASDISLLDAQ |
| SEQ ID NO: 69 | AA 6-32 | TMKGLDIQKVAGTWYSLAMAASDISLL |
| SEQ ID NO: 70 | AA 1-32 | XIVTQTMKGLDIQKVAGTWYSLAMAASDISLL |

| | | |
|---|---|---|
| SEQ ID NO: 71 | AA 13-30 | QKVAGTWYSLAMAASDIS |
| SEQ ID NO: 72 | AA 19-36 | WYSLAMAASDISLLDAQS |
| SEQ ID NO: 73 | AA 25-42 | AASDISLLDAQSAPLRVY |
| SEQ ID NO: 74 | AA 31-48 | LLDAQSAPLRVYVEELKP |

Herein, X defines an amino acid selected from leucine (L) or isoleucine (I), preferably X is leucine. The choice for X being either leucine or isoleucine is a direct consequence of the source of mammalian milk. Leucine is preferred, since mammalian milk from the genus Bos, preferably cow's milk, is most preferred.

In a preferred embodiment of the present invention, the ultrafiltered whey protein hydrolysate comprises, at least 10 microgram per gram of protein, more preferably at least 50 microgram per gram protein, even more preferably at least 100 microgram per gram protein of the sum of betalactoglobulin peptides comprising an amino acid sequence according to any one of SEQ ID No. 2 - 70, preferably according to any one of SEQ ID No. 31-70. Preferably the ultrafiltered whey protein hydrolysate comprises10 to 50,000 microgram per gram protein, more preferably 20 to 20000 microgram per gram protein, more suitable 30 to 10,000 microgram per gram protein and particularly preferably 50 to 5,000 microgram per gram protein of the sum of betalactoglobulin peptides comprising an amino acid sequence according to any one of SEQ ID No. 2 - 70, preferably according to any one of SEQ ID No. 31-70. The betalactoglobulin derived peptides are preferably present in therapeutically effective amounts.

In a preferred embodiment of the present invention, the ultrafiltered whey protein hydrolysate comprises, at least 10 microgram per gram of protein, more preferably at least 50 microgram per gram protein, even more preferably at least 100 microgram per gram protein of the sum of betalactoglobulin peptides comprising an amino acid sequence with a length of 8 amino acids according to any one SEQ ID no. 2-30, more preferably an amino acid sequence with a length of 10 amino acids according to any one SEQ ID no. 31-57.

### Allergic betalactoglobulin

Preferably the ultrafiltered whey protein hydrolysate comprises less than 6 microgram allergenic betalactoglobulin per g protein, more preferably less than 0.8 microgram allergenic betalactoglobulin per g protein, most preferably less than 0.2 microgram per g protein. In the context of the invention, the expression `allergenic betalactoglobulin' refers to intact or immunogenic betalactoglobulin, and does not account for the hydrolyzed betalactoglobulin. For sake of comparison, an ultrafiltered hydrolyzed whey protein exhibits less than 0.2 microgram allergenic betalactoglobulin per g protein. Allergenic betalactoglobulin can be determined by methods as known in the art, such as Elisa.

### Emulsifier

Food emulsifiers act as an interface between the conflicting components of food like water and oil. To make the two components compatible, emulsifiers are used. Not bound by the theory, the inventors speculate emulsifiers may prevent clustering of tolerogenic peptides or break up clusters oftolerogenic peptides, enabling these peptides to permeate the ultrafiltration membrane. Examples of emulsifiers are phospholipids, magnesium stearate and sodium, potassium and calcium salts of fatty acids. Further examples are lecithin's such as soy lecithin, calcium stearoyl-2-lactylate (CSL), polyglycerol esters (PGE), sorbitan esters (SOE) such as sorbitan monostearate, propylene glycol monoesters (PGME), sucrose esters (SE / E473), monoglycerides/monoacylglycerols (MAG), diglycerides/diacylglycerols (DAG), phosphated monoglycerides, acetylated monoglycerides (AMG), lactylated monoglycerides (LMG), diacetyl tartaric acid esters of mono- and diglycerides (Datem), citric acid esters of mono and diglycerides (Citrem). A criterion is that the food emulsifier should be suited for use in infant and follow-on formula. In the context of the current invention preferably the emulsifier is selected from the group consisting of Citrem, Datem, MAG, DAG, SE (E473) and soy lecithin, and combinations thereof, more preferably is or comprises Citrem. Preferably the ultrafiltered whey protein hydrolysate comprises an emulsifier selected from the group consisting of Citrem, Datem, MAG, DAG SE (E473) and soy lecithin, and combinations thereof, more preferably is or comprises Citrem.

Preferably the emulsifier is added in the process for preparing an ultrafiltered whey protein hydrolysate in a concentration of at least 50 mg per gram protein to which it is added, more preferably at least 100 mg per gram protein, most preferably 150 - 500 mg per gram protein. Preferably the emulsifier is added in the process for preparing an ultrafiltered whey protein hydrolysate in a concentration of at least 1 g per liter, more preferably at least 2 g per liter, most preferably 2- 20 g per liter, particularly 5 - 15 g per liter.

Preferably the emulsifier is added in the process for preparing an ultrafiltered whey protein hydrolysate prior to hydrolysis and/or during hydrolysis, more preferably prior to hydrolysis.

### Chaotropic agent

In one embodiment a chaotropic agent is used as a component to ensure the tolerogenic betalactoglobuline peptides pass the membrane filter. A chaotropic agent is a substance which disrupts the structure of and denatures, macromolecules such as proteins. Common chaotropic agents suitable in the manufacturing process of the invention include n-butanol, ethanol, guanidinium chloride, lithium perchlorate, lithium acetate, magnesium chloride, phenol, 2-propanol, thiourea, SDS and urea. For the current invention, preferably the chaotropic agent is or comprises urea. Chaotropic agents may solubilize the peptide aggregates after the hydrolysis and/or prevent the formation of peptide aggregates.

As chaotropic agents may have a denaturing effect on all proteins (including proteolytic enzymes), the chaotropic agent is preferably added only after hydrolysis 1.

The ultrafiltered whey protein hydrolysate and/or the process for preparing an ultrafiltered whey protein hydrolysate according to the present invention preferably comprises urea as chaotropic agent, marking the use of urea in the manufacturing process. Urea is also known as carbamide, CO(NH2)2. Urea is commercially available from various sources in food-grade quality, for example from NuGenTec, CA USA, Spectrum chemical, USA, or it can be enriched from cow's milk as disclosed in US 6,506,305.

According to the present invention, in one embodiment the chaotropic agent is used in the process of the invention in an amount of at least 10 g chaotropic agent per 100 ml, more preferably 20 - 100 g chaotropic agent per 100 ml, preferably urea. Preferably, the chaotropic agent is provided in the process of the invention in an amount of at least 5 g chaotropic agent per gram protein to which it is added, preferably 5 - 20 g per gram protein. The ultrafiltered whey protein hydrolysate preferably comprises 1 to 25 g chaotropic agent (preferably urea) per gram protein, more preferably 5 to 20 g per gram protein, more preferably 7 to 15 g per gram protein. Preferably, the ultrafiltered whey protein hydrolysate comprises at least 1 g per 100 ml, more preferably 5 to 80 g chaotropic agent per 100 ml, most preferably 20 to 60 g chaotropic agent per 100 ml.

According to the present invention, in another preferred embodiment the chaotropic agent is used in the process of the invention in an amount of at least 1 g chaotropic agent (preferably urea) per 100 ml, more preferably of at least 3 g chaotropic agent per 100 ml, even more preferably 1 - 10 g chaotropic agent per 100 ml, preferably urea. Preferably, the chaotropic agent is provided in the process of the invention in an amount of at least 25 mg chaotropic agent per gram protein to which it is added, preferably 50 - 200 mg per gram protein. Preferably the ultrafiltered whey protein hydrolysate comprises 10 to 250 mg chaotropic agent (preferably urea) per gram protein, more preferably 20 to 200 mg per gram protein, more preferably 50 to 150 mg per gram protein. Alternatively or additionally, the ultrafiltered whey protein hydrolysate comprises at least 10 mg chaotropic agent (preferably urea) per gram dry weight, preferably at least 50 mg per gram dry weight, more preferably at least 100 mg per gram dry weight. In a further embodiment according to the present invention, the ultrafiltered whey protein hydrolysate comprises at least 15 mg per 100 ml, more preferably 15 to 75 mg chaotropic agent per 100 ml.

### Lactic acid producing bacteria

The nutritional composition according to the invention preferably comprises a strain of lactic acid producing bacterium species, which enhances the oral immune tolerogenic capacity of the betalactoglobulin peptides of the invention. The bacterium strain is preferably a probiotic. Evidence of synergy is attached in the examples. The presence of lactic acid producing bacteria was found to increase the expression of HLA-DR molecules on the surface of the dendritic cells. An increased expression of HLA-DR is also indicative for an improved presentation of peptides to T cells, and hence oral immune tolerance induction and is indicative for treatment and/or prevention of allergy as defined herein. Suitable lactic acid producing bacteria include strains of the genus *Bifidobacteria* (e.g. *B. breve, B. longum, B. infantis, B. bifidum), Lactobacillus* (e.g. *L. acidophilus, L. paracasei, L. johnsonii, L. plantarum, L. reuteri, L. rhamnosus, L. casei, L. lactis),* and *Streptococcus* (e.g. *S*. *thermophilus*)*. Bifidobacterium breve* and *Bifidobacterium longum* are especially suitable lactic acid producing bacteria.

The nutritional composition according to the invention preferably comprises a strain of lactic acid-producing bacterium belonging to the genus *Bifidobacterium,* preferably to the species *Bifidobacterium breve.* The B. *breve* preferably has at least 95 % identity of the 16 S rRNA sequence when compared to the type strain of *B. breve* ATCC 15700, more preferably at least 97% identity (Stackebrandt & Goebel, 1994, Int. J. Syst. Bacteriol. 44:846-849). Suitable *B. breve* strains may be isolated from the faeces of healthy human milk-fed infants. Typically, these are commercially available from producers of lactic acid bacteria, but they can also be directly isolated from faeces, identified, characterised and produced. According to one embodiment, the nutritional composition contains a B. *breve* selected from the group consisting of B. *breve* Bb-03 (Rhodia/Danisco), *B. breve* M-16V (Morinaga), *B. breve* R0070 (Institute Rosell, Lallemand), *B. breve* BR03 (Probiotical), *B. breve* BR92) (Cell Biotech), DSM 20091, LMG 11613, YIT4065, FERM BP-6223 and CNCM I-2219. B. *breve* can be B. *breve* M-16V and B. *breve* CNCM I-2219, most preferably B. *breve* M-16V. *B. breve* I-2219 was published in WO 2004/093899 and was deposited at the Collection Nationale de Cultures de Microorganisms, Institute Pasteur, Paris, France on 31 May 1999 by Compagnie Gervais Danone. B. *breve* M-16V was deposited as BCCM/LMG23729 and is commercially available from Morinaga Milk Industry Co., Ltd.

The lactic acid producing bacterium may be present in the nutritional composition at any suitable concentration, preferably in a therapeutically effective amount or "amount effective for treating/prevention" in the context of the invention. Preferably, the lactic acid producing bacterium strain is included in the nutritional composition in an amount of 10⁴ - 10¹³ cfu per g dry weight of the nutritional composition, preferably 10⁵ - 10¹¹ cfu/g, most preferably 10⁶ - 10¹⁰ cfu/g.

### Non-digestible oligosaccharides

In a preferred embodiment, the nutritional composition of the invention additionally comprises one or more non-digestible oligosaccharides [NDO]. Like the lactic acid-producing bacteria, these further increase the oral immune tolerance inducing properties of peptides of the betalactoglobulin protein and improve treatment and/or prevention of allergy as defined herein. Evidence of an enhanced effect is given in PCT/NL2019/050237 and in the experimental section.

Advantageously and most preferred, the non-digestible oligosaccharide is water-soluble (according to the method disclosed in L. Prosky et al, J. Assoc. Anal. Chem 71: 1017-1023, 1988) and is preferably an oligosaccharide with a degree of polymerisation (DP) of 2 to 200. The average DP of the non-digestible oligosaccharide is preferably below 200, more preferably below 100, even more preferably below 60, most preferably below 40.

The non-digestible oligosaccharide is preferably a prebiotic. It is not digested in the intestine by the action of digestive enzymes present in the human upper digestive tract (small intestine and stomach). The non-digestible oligosaccharide is fermented by the human intestinal microbiota. For example, glucose, fructose, galactose, sucrose, lactose, maltose and the maltodextrins are considered digestible. The oligosaccharide raw materials may comprise monosaccharides such as glucose, fructose, fucose, galactose, rhamnose, xylose, glucuronic acid, GalNac etc., but these are not part of the oligosaccharides. The non-digestible oligosaccharide is preferably selected from the group consisting of fructooligosaccharide, non-digestible dextrin, galactooligosaccharide, xylooligosaccharide, arabino-oligosaccharide, arabinogalacto-oligosaccharide, gluco-oligosaccharide, glucomanno-oligosaccharide, galactomanno-oligosaccharide, mannan-oligosaccharide, chito-oligosaccharide, uronic acid oligosaccharide, sialyloligosaccharide and fucooligosaccharide, and mixtures thereof, preferably fructo-oligosaccharides. Examples of sialyloligosaccharide are 3-sialyllactose, 6" sialyllactose, sialyllacto-N-tetraoses, disialyllactoNtertraoses. Examples of fucooligosaccharides are (un)sulphated fucoidan oligosaccharides, 2'fucosyllactose, 3' fucosyllactose, lacto-N-fucopentaose I, II, III, LNDH, lactodifucotetraose, lacto-N difucohexaose I and II.

One suitable type of oligosaccharide is a short-chain oligosaccharide which has an average degree of polymerisation of less than 10, preferably at most 8, preferably in the range of 2 - 7. The short-chain oligosaccharide preferably comprises galacto-oligosaccharides and/or fructo-oligosaccharides (i.e. scGOS and/or scFOS). In one embodiment, the nutritional composition comprises galacto-oligosaccharides, preferablybeta-galacto-oligosaccharides, preferably trans-galacto-oligosaccharides. The galacto-oligosaccharides preferably have an average degree of polymerisation in the range of 2 - 8, preferably 3 - 7, i.e. are short-chain oligosaccharides in the context of the invention. (Trans)galactooligosaccharides are for example available under the trade name Vivinal^{®}GOS (Friesland Campina Domo Ingredients, Netherlands), Bimuno (Clasado), Cup-oligo (Nissin Sugar) and Oligomate55 (Yakult). The nutritional composition preferably comprises short-chain fructo-oligosaccharides and/or short-chain galacto-oligosaccharides, preferably at least short-chain fructo-oligosaccharides. Fructooligosaccharides may be inulin hydrolysate products having an average DP within the aforementioned (sub-) ranges; such FOS products are for instance commercially available as Raftilose P95 (Orafti) or with Cosucra.

Another suitable type of oligosaccharide is long-chain fructo-oligosaccharides (IcFOS) which has an average degree of polymerisation above 10, typically in the range of 10 - 100, preferably 15 - 50, most preferably above 20. A particular type of long-chain fructo-oligosaccharides is inulin, such as Raftilin HP.

The nutritional composition may contain a mixture of two or more types of non-digestible oligosaccharides, most preferably a mixture of two non-digestible oligosaccharides. In case the NDO comprises or consists of a mixture of two distinct oligosaccharides, one oligosaccharide may be short-chain as defined above and one oligosaccharide may be long-chain as defined above. Most preferably, short-chain oligosaccharides and long-chain oligosaccharides are present in a weight ratio short-chain to long-chain in the range of 1:99 - 99:1, more preferably 1:1 - 99:1, more preferably 4:1 - 97:3, even more preferably 5:1 - 95:5, even more preferably 7:1 - 95:5, even more preferably 8:1 - 10:1, most preferably about 9:1.

In one embodiment, the nutritional composition comprises at least two of fructo-oligosaccharides and/or galacto-oligosaccharides. Suitable mixtures include mixtures of long-chain fructo-oligosaccharides with short-chain fructo-oligosaccharides or with short-chain galacto-oligosaccharides, most preferably long-chain fructo-oligosaccharides with short-chain fructo-oligosaccharides.

The nutritional composition preferably comprises 0.05 to 20 wt% of said non-digestible oligosaccharides, more preferably 0.5 to 15 wt%, even more preferably 1 to 10 wt%, most preferably 2 to 10 wt%, based on dry weight of the nutritional composition. When in liquid form, the nutritional composition preferably comprises 0.01 to 2.5 wt% non-digestible oligosaccharide, more preferably 0.05 to 1.5 wt%, even more preferably 0.25 to 1.5 wt%, most preferably 0.5 - 1.25 wt%, based on 100 ml.

In one embodiment, the NDO mixture does not comprises any detectable amounts of acid oligosaccharides.

When the non-digestible oligosaccharide is a mixture, the averages of the respective parameters are used for defining the present invention.

The combination of a NDO and a lactic acid producing bacterium as defined here above is also referred to as a "synbiotic". The presence of therapeutically effective amounts of the NDO together with the lactic acid-producing bacterium are believed to further improve the oral immune tolerance inducing properties and the effect on treatment and/or prevention of allergy of a strain of *Bifidobacterium,* preferably B. *breve,* together with fructo-oligosaccharides.

### Other components

The nutritional composition of the current invention may further comprise long chain polyunsaturated fatty acids (LC-PUFA). LC-PUFA are fatty acids wherein the acyl chain has a length of 20 to 24 carbon atoms (preferably 20 or 22 carbon atoms) and wherein the acyl chain comprises at least two unsaturated bonds between said carbon atoms in the acyl chain. More preferably the nutritional composition comprises at least one LC-PUFA selected from the group consisting of eicosapentaenoic acid (EPA, 20:5 n3), docosahexaenoic acid (DHA, 22:6 n3), arachidonic acid (ARA, 20:4 n6) and docosapentaenoic acid (DPA, 22:5 n3), preferably DHA, EPA and/or ARA. Such LC-PUFAs have a further beneficial effect on reducing the risk for allergy.

The preferred content of LC-PUFA in the nutritional composition does not exceed 15 wt.% of total fatty acids, preferably does not exceed 10 wt.%, even more preferably does not exceed 5 wt.%. Preferably the nutritional composition comprises at least 0.2 wt.%, preferably at least 0.25 wt.%, more preferably at least 0.35 wt.%, even more preferably at least 0.5 wt.% LC-PUFA of total fatty acids, more preferably DHA. The nutritional composition preferably comprises ARA and DHA, wherein the weight ratio ARA/DHA preferably is above 0.25, preferably above 0.5, more preferably 0.75 - 2, even more preferably 0.75-1.25. The weight ratio is preferably below 20, more preferably between 0.5 and 5. The amount of DHA is preferably above 0.2 wt%, more preferably above 0.3 wt%, more preferably at least 0.35 wt%, even more preferably 0.35 - 0.6 wt% on total fatty acids.

### Nutritional composition

The nutritional composition according to the invention can be used as a nutritional therapy, nutritional support, as a medical food, as a food for special medical purposes or as a nutritional supplement. The nutritional composition is preferably an enteral (oral) composition. The nutritional composition is administered orally to, or intended to be administered orally to, a subject in need thereof, in particular to children and infants, including toddlers, preferably children up to 6 years of age, preferably infants or young children typically with an age of 0 - 36 months, more preferably infants 0-12 months of age, most preferably 0-6 months of age. Thus, in some embodiments, the nutritional composition is an infant formula, follow-on formula or young child formula (also referred to as growing-up milk), preferably it is an infant formula or follow-on formula, most preferably an infant formula. The terms 'infant formula' and 'follow-on formula' are well-defined and controlled internationally and consistently by regulatory bodies. In particular, CODEX STAN 73 - 1981 "Standard For Infant Formula and Formulas For Special Medical Purposes Intended for Infants*"* is widely accepted. It recommends for nutritional value and formula composition, which require the prepared milk to contain per 100 ml not less than 60 kcal (250 kJ) and no more than 70 kcal (295 kJ) of energy. FDA and other regulatory bodies have set nutrient requirements in accordance therewith.

Preferably, the nutritional composition of the invention is for providing the daily nutritional requirements to a human, in particular for administration to, in particular for feeding, humans, in particular infants including toddlers, preferably at risk for developing bovine, ovine and/or caprine milk protein allergy, preferably against milk protein from a species of the genus *Bos, Bison, Bubalus* or *Capra,* more preferably from genus Bos, most preferably cow's milk protein allergy. In one embodiment, the milk protein allergy concerns milk from a particular mammalian species and the ultrafiltered whey protein hydrolysate comprised in the nutritional composition is from the same mammalian species, preferably both are from the genus Bos.

In order to meet the caloric requirements of the infant, the nutritional composition of the invention preferably comprises 50 to 200 kcal/100 ml liquid, more preferably 60 to 90 kcal/100 ml liquid, even more preferably 60 to 75 kcal/100 ml liquid. This caloric density ensures an optimal ratio between water and calorie consumption. The osmolarity of the nutritional composition is preferably between 150 and 420 mOsmol/l, more preferably 260 to 320 mOsmol/l. The low osmolarity aims to reduce the gastrointestinal stress.

Preferably, the nutritional composition of the invention is in a liquid form, preferably with a viscosity below 35 mPa.s, more preferably below 6 mPa.s as measured in a Brookfield viscometer at 20°C at a shear rate of 100 s-1. Suitably, the nutritional composition is in a powdered form, which preferably can be reconstituted with water to form a liquid, or in a liquid concentrate form, which should be diluted with water. When the nutritional composition is in a liquid form, the preferred volume administered on a daily basis is in the range of about 80 to 2500 ml, more preferably about 450 to 1000 ml per day.

The nutritional composition according to the invention comprises a lipid component, preferably a lipid component suitable for infant nutrition as known in the art. The lipid component of the nutritional composition preferably provides 2.9 to 6.0 g, more preferably 4 to 6 g per 100 kcal of the nutritional composition. When in liquid form, the nutritional composition preferably comprises 2.1 to 6.5 g lipid per 100 ml, more preferably 3.0 to 4.0 g per 100 ml. Based on dry weight the present infant or follow-on formula preferably comprises 12.5 to 40 wt% lipid, more preferably 19 to 30 wt%.

The nutritional composition according to the invention may comprise further proteinaceous material, in addition to the beta-lactoglobulin-derived peptide(s) according to the invention. In the context of the present invention the additional "protein" or "proteinaceous material" or "protein equivalents" encompasses proteins, peptides, free amino acids and partially or extensively hydrolysed proteins. Preferably, the further proteinaceous material - additional to the ultrafiltered whey protein hydrolysate - does not evoke an allergic reaction or is hypoallergenic, such as free amino acids, and hydrolysed protein. As a further protein component, i.e. apart from the ultrafiltered whey protein hydrolysate, the nutritional composition according to the present invention may comprise free amino acids. The nutritional composition according to the present invention preferably contains less than 1 wt% intact mammalian (cow)'s milk protein. The nutritional composition may comprise an additional protein component selected from the group consisting of free amino acids and proteins from other sources such as soy, pea, rice, collagen or the like, in intact form, in partially hydrolysed form, and/or in extensively hydrolysed form. In a most preferred embodiment, the composition is free from protein component other than the ultrafiltered whey protein hydrolysate. That is, it is preferred that the ultrafiltered whey protein hydrolysate is the sole source of proteinaceous material in the composition.

The nutritional composition preferably contains 4 to 25 %, more preferably 5 to 20 %, more preferably 7 to 16 %, most preferably 7 to 12 % protein, based on total calories. The nutritional composition, when in liquid form, preferably contains 0.5 to 6.0 g, more preferably 0.8 to 3.0 g, even more preferably 1.0 to 2.5 g of protein per 100 ml. The nutritional composition preferably comprises at least 7.0 wt%, more preferably at least 8.0 wt%, most preferably at least 9 or at least 10 wt% protein based on dry weight of the total composition. Preferably, the nutritional composition comprises at most 40 wt%, more preferably at most 15 wt%, preferably at most 20 wt% of protein based on dry weight of the total composition.

The nutritional composition comprises digestible carbohydrate(s). Typically, digestible carbohydrates that are known in the art to be suitable for use in infant nutritional compositions are used, for example selected from digestible polysaccharides (e.g. starch, maltodextrin), digestible monosaccharides (e.g. glucose, fructose), and digestible disaccharides (e.g. lactose, sucrose). Particularly suitable is lactose and/or maltodextrin. In one embodiment, the nutritional composition does not comprise lactose. The digestible carbohydrate component preferably comprises at least 60 wt% lactose based on total digestible carbohydrate, more preferably at least 75 wt%, even more preferably at least 90 wt% lactose based on total digestible carbohydrate.

### Human subjects

The human subjects or population targeted is children and infants, including toddlers, preferably children up to 6 years of age, preferably infants or young children typically with an age of 0 - 36 months, more preferably infants 0-12 months of age, most preferably 0 - 6 months of age. In one preferred embodiment, the subject is at risk of developing allergy, preferably food allergy, preferably bovine, ovine and/or caprine milk protein allergy, preferably milk protein from a species of the genus *Bos, Bison, Bubalus* or *Capra,* more preferably from genus Bos, most preferably cow's milk protein allergy, preferably infants suffering from milk protein allergy preferably against milk protein from a species of the genus *Bos, Bison, Bubalus* or *Capra,* more preferably from genus Bos, most preferably cow's milk protein allergy. Infants at risk are preferably determined by the family history, preferably at least one parent being or having a history of being allergic. In one aspect, the invention thus relates to the primary prevention of allergy as defined here above.

In a more preferred embodiment, the invention relates to the secondary prevention of allergy as defined here above.

In one aspect, the subject suffers from allergy, preferably food allergy, preferably bovine, ovine and/or caprine milk protein allergy, preferably milk protein from a species of the genus *Bos, Bison, Bubalus* or *Capra,* more preferably from genus Bos, most preferably cow's milk protein allergy, preferably infants suffering from milk protein allergy preferably against milk protein from a species of the genus *Bos, Bison, Bubalus* or *Capra,* more preferably from genus Bos, most preferably cow's milk protein allergy.

The invention pertains to the use of the ultrafiltered whey protein hydrolysate or a composition comprising said ultrafiltered whey protein hydrolysate according to the invention in the manufacture of a product for:
(i) providing nutrition to; and/or
(ii) treating/preventing allergy as defined here above in; and/or
(iii) inducing oral immune tolerance against an allergen, preferably a food allergen, in
subjects as defined here above. The use is preferably directed to (ii) and/or (iii).

The invention also pertains to ultrafiltered whey protein hydrolysate or a composition comprising said ultrafiltered whey protein hydrolysate as defined here above, for use in:
(i) providing nutrition to; and/or
(ii) treating/preventing allergy as defined here above in; and/or
(iii) inducing oral immune tolerance against an allergen, preferably a food allergen, in
subjects as defined here above. The use is preferably directed to (ii) and/or (iii).

Worded differently, described herein is also a method for:
(i) providing nutrition to; and/or
(ii) treating/preventing allergy as defined here above in; and/or
(iii) inducing oral immune tolerance against an allergen, preferably a food allergen, in
subjects as defined here above, wherein said subject is administered ultrafiltered whey protein hydrolysate or a composition comprising said ultrafiltered whey protein hydrolysate. The method is preferably directed to (ii) and/or (iii). In case of a method for (i) providing nutrition, the method is preferably a non-therapeutic method.

### EXAMPLES

### Example 1: A membrane filtration step of hydrolysed whey protein results in retention of tolerogenic betalactoglobulin peptides.

In a Multifors reactor (Infors HT) 500 gram of whey protein powder (WPC83, Arla Foods, with about 80% whey protein) was dissolved in water at a final concentration of about 4 wt% at a temperature of about 75°C, giving a protein concentration of about 3.2 wt%. After dissolving the mixture was cooled to 58°C and the pH was adjusted to neutral (pH 7-8). Proteolytic enzymes were added (Trypsin, Neova Technologies Inc) to a concentration of 33mg/100g solution and hydrolysis took place for about 105 min under continuous stirring. During hydrolysis the pH was kept constant. After hydrolysis the mixture was quickly cooled to 4°C.

Next the mixture of whey protein hydrolysate and proteolytic enzymes was filtrated. 200ml of the mixture (the feed) was filtrated for 20 minutes on a MMS Triple System membrane filtration unit (MMS AG Membrane Systems) provided with an ultrafiltration membrane from Koch (Koch: HFK-131, a flat sheet polyethersulfone ultrafiltration membrane with a molecular weight cut off of 10 kDa) under a pressure of 8 bars. A one-layer filtration setup was used and the retentate was circulated during filtration. Each membrane was used only once to promote reproducibility. The filtration cooling system was set to 15°C. The permeate was weighted, MMS filtration software was used for the registering of the permeate throughput. Samples were taken of the mixture before membrane filtration (the feed), and of the permeate and retentate after the membrane filtration step. About 93% of permeate (wt% of total feed) was produced comprising about 85wt% of peptides (wt% of the peptides in the feed). Samples were stored at -50°C for further analysis.

The presence of tolerogenic betalactoglobulin peptides in the samples was analyzed according to Gouw et al., 2018, Clin. Exp. Allergy 48:1345-1253, with the modification that instead of nanoLC-ESI MS2, micro LC-MS ESI MS2 was used. The presence of 5 specific peptides was tested (region overlapping with the 13-48 AA acid region of betalactoglobulin in bold):
Peptide 1: AA 1-27;
   **LlVTQTMKG LDIQKV AGTWYSLAMAAS**
Peptide 2: AA 11-42;
   **DIQKVAGTWYSLAMAASDISLLDAQSAPLRVY**
Peptide 3: AA 6-35;
   **TMKGLDIQKVAGTWYSLAMAASDISLLDAQ**
Peptide 4: AA 6-32;
   **TMKGLDIQKVAGTWYSLAMAASDISLL**
Peptide 5: AA 1-32;
   LIVTQTMKGLDI**QKVAGTWYSLAMAASDISLL**

These peptides correspond to SEQ ID NOs 66 - 70, respectively, with X being L.

It was found that these betalactoglobulin peptides were detected in the hydrolysate mixture before the membrane filtration step and in the retentate, but not in the permeate. As a result of the membrane filtration step, the amount of allergic beta-lactoglobulin in the permeate was lower than 0.2 microgram/gram protein and the amount of peptides larger than 10kDa in the permeate was lower than 1 mg/gram protein. Both parameters can be determined by methods known in the art (ELISA and size exclusion high pressure liquid chromatography).

### Example 2a: Presence of emulsifier during hydrolysis results in the presence of betalactoglobulin peptides in the permeate after the membrane filtration step.

The process of example 1 was repeated. The only difference was that Citrem (Grinsted^{®} Citrem, DuPont) was added at an amount of about 3.6 gram before the pH adjustment and the addition of the proteolytic enzymes. Citrem is not retained by the UF-membrane and therefore the Citrem concentration in permetate and retentate will be similar. Moreover, Citrem addition was found not to have an effect on retention of peptides larger than 10 kDa and of allergic beta-lactoglobulin. As for example 1, the feed, retentate and permeate was tested for tolerogenic peptides. Surprisingly and contrary to example 1, all of the 5 tolerogenic peptides (peptides 1-5 of example 1 (i.e. SEQ ID NO 66 - 70 with X = L) were detected in the permeate, in an amount of about 1-10 wt.% of the concentration of these peptides in the feed.

### Example 2b: The experiment of example 2a was repeated but the Citrem was added in the middle of the hydrolysis step instead, and again the specific peptides were now found in the permeate.

### Example 3: Presence of chaotropic agent during hydrolysis results in the presence of betalactoglobulin peptides in the permeate after the membrane filtration step.

The process of example 2a was repeated, the only difference being that instead of the emulsifier, 51 gram of urea (source Merck) was dissolved per 100 g water (8 M) and added after the hydrolysis step and before ultrafiltration. Urea is not retained by the UF-membrane and therefore the urea concentration in permeate and retentate will be similar. Moreover, urea addition was not found to have an effect on retention of peptides larger than 10 kDa and of allergic beta-lactoglobulin. The urea was dissolved just before the membrane filtration step and allowed to cool. The cooling time before filtration was found sufficient time. The tolerogenic betalactoglobulin peptides were found to be present in the feed and retentate but surprisingly also in the permeate. All peptides were detected in the retentate and feed, and peptides 1, 3, 4 and 5 of example 1 (i.e. SEQ ID NOs 66, 68, 69 and 70 with X = L) were detected in the permeate, in an amount of about 1-10 wt.% of the concentration of these peptides in the feed.

### Example 3: Presence of chaotropic agent during hydrolysis results in the presence of betalactoglobulin peptides in the permeate after the membrane filtration step.

The process of example 2a was repeated, the only difference being that instead of the emulsifier, 51 gram of urea (source Merck) was dissolved in 100 g water (8 M) and added after the hydrolysis step and before ultrafiltration. Urea is not retained by the UF-membrane and therefore the urea concentration in permeate and retentate will be similar. Moreover, urea addition was not found to have an effect on retention of peptides larger than 10 kDa and of allergic beta-lactoglobulin. The tolerogenic betalactoglobulin peptides were found to be present in the feed and retentate but surprisingly also in the permeate. All peptides were detected in the retentate and feed, and peptides 1, 3, 4 and 5 of example 1 (i.e. SEQ ID NOs 66, 68, 69 and 70 with X = L) were detected in the permeate, in an amount of about 1-10 wt.% of the concentration of these peptides in the feed.

### Example 4: Infant formula

A powdered Infant Formula intended for infants of 0-6 months of age that suffer from food allergy, comprising 66 kcal per 100 ml when reconstituted with water to ready-to-drink liquid,
- comprising about 2.4 g protein per 100 kcal,
- the protein being an ultrafiltered, hydrolyzed whey protein obtainable by the process described in example 2, comprising at least 3 of the betalactoglobulin peptides selected from the group consisting of
LIVTQTMKGLDIQKVAGTWYSLAMAAS,
DIQKVAGTWYSLAMAASDISLLDAQSAPLRVY,
TMKGLDIQKVAGTWYSLAMAASDISLLDAQ,
TMKGLDIQKVAGTWYSLAMAASDISLL, and
LIVTQTMKGLDIQKVAGTWYSLAMAASDISLL,
and with a MW distribution pattern of the peptides in the whey protein hydrolysate of

| | |
|---|---|
| < 1 kDa | 72.5-88.7 % |
| 1-5 kDa | 15.5-22.9 % |
| 5-10 kDa | 0-0.1 % |
| >10 kDa | 0-0.1% |

The infant formula further comprises 10.7 g digestible carbohydrates (mainly lactose) per 100 kcal, 5.1 g fat per 100 kcal, 0.8 g non-digestible oligosaccharides per 100 ml (scGOS/IcFOS in a 9:1 ratio), citrem, vitamins, minerals and trace elements as known in the art.

### Example 5: Infant formula

A powdered Infant Formula intended for infants of 0-12 month that suffer from food allergy, comprising 66 kcal per 100 ml when reconstituted with water to ready to drink liquid,
- comprising about 2.7 g protein per 100 kcal,
- the protein being an ultrafiltered, hydrolyzed whey protein obtainable by the process described in example 3, comprising at least 3 of the betalactoglobulin peptides selected from the group consisting of
   LIVTQTMKGLDIQKVAGTWYSLAMAAS,
   DIQKVAGTWYSLAMAASDISLLDAQSAPLRVY,
   TMKGLDIQKVAGTWYSLAMAASDISLLDAQ,
   TMKGLDIQKVAGTWYSLAMAASDISLL, and
   LIVTQTMKGLDIQKVAGTWYSLAMAASDISLL,
and with a MW distribution pattern of the peptides in the whey protein hydrolysate of

| | |
|---|---|
| < 1 kDa | 62.8-74.6 % |
| 1-5 kDa | 27.9-34.8 % |
| 5-10 kDa | 0-0.3% |
| >10 kDa | 0-0.1% |

The infant formula further comprises 10.9 g digestible carbohydrates (mainly lactose), 5.1 g fat per 100 kcal, 0.8 g non digestible oligosaccharides per 100 ml (scGOS/lcFOS in a 9:1 ratio), urea, vitamins, minerals and trace elements as known in the art.

### Example 6: Synbiotic mixture shows a further improved effect of tolerance induction compared to probiotics or prebiotics alone

Several published studies support a notion that dietary antigens (including peptides) within the intestinal lumen can be absorbed and subsequently captured and presented by intestinal antigen-presenting cells, i.e., antigen sampling mechanisms, without the need of a disrupted intestinal barrier. There are at least two distinct mechanisms that continuously work to sample dietary antigens at the healthy state, i.e., microfold /M cell-mediated transcytosis and goblet-cell-associated antigen passage. Both pathways provide dietary antigens to intestinal lamina propria CD103+ DCs, which in return could imprint gut-homing molecules on T and B cells, could promote differentiation of intestinal IgA-producing plasma cells as well as could generate intestinal Tregs, a key player in the state of hyporesponsiveness to fed antigen known as oral tolerance. This suggests that peptides within the tested HP can be orally absorbed, followed by antigen capture and presentation by intestinal CD103+ DCs and subsequently culminated in generation of functional Tregs.

For the development of oral tolerance, T-cell epitopes within the tested HP need to be presented under the right circumstances. In addition to TCR activation, co-stimulation and cytokine signalling play an important role in the generation of Tregs. Pro- or prebiotics play an important role in creating the right environment for this predisposition towards Tregs. The preventive effect of synthetic BLG peptides was strengthened in combination with a probiotic or prebiotic diet.

To demonstrate the effect of pro-, pre- and synbiotics an experiment was performed similar as described in example 4 of WO 2016/148572, with the same protocol, and the same concentration and mixture of synthetic peptides ('Pepmix'), the pepmix identified in Table 8.

**Table 8.**

| **pepmix according to** WO 2016/148572 | | |
|---|---|---|
| Peptide | | Sequence |
| 1 (18AA) | SEQ ID NO: 71 | QKVAGTWYSLAMAASDIS |
| 2 (18AA) | SEQ ID NO: 72 | WYSLAMAASDISLLDAQS |
| 3 (18AA) | SEQ ID NO: 73 | AASDISLLDAQSAPLRVY |
| 4 (18AA) | SEQ ID NO: 74 | LLDAQSAPLRVYVEELKP |

A number of diets were compared:
(a) synbiotic diet of example 1 of WO 2016/14472 (1 wt% scFOS/lcFOS in a wt ratio 9:1 + 2 wt% 2×10⁹ cfu/g *Bifidobacterium breve* M-16V,
(b) probiotic diet of example 4 of WO 2016/148572 with 2 wt% 2×10⁹ cfu/g *Bifidobacterium breve* M-16V, and
(c) prebiotic diet containing 1 wt% of scFOS/lcFOS wt ratio 9/1.

The short-chain (sc-) and long-chain (Ic-) fructo-oligosaccharides (FOS) were commercially available as Raftilose P95 (Orafti) and Raftiline HP, respectively.

All groups were pretreated with the pepmix and one of the above diets, were sensitized with whey + cholera toxin and challenged with whey.

After challenge with whey, the delta ear swelling of the mice having consumed (c) scFOS/lcFOS diet was about 174 µm, of the mice having consumed (b) the probiotic diet was about 158 µm, and of the mice having consumed (a) the synbiotic diet was about 112 µm. The ear swelling in the synbiotic group
(a) was statistically significantly lower than in the corresponding scFOS/lcFOS group (c), and there was a trend of a lower response when compared with probiotic group (b) (p=0.08). These results are indicative for a further improved effect on oral immunotolerance induction:
   - when using a lactic acid-producing bacterium, in particular a *Bifidobacterium* strain from the species *B. breve,* and
   - particularly when using a lactic-acid producing bacterium in combination with NDOs.

Based on these experiments, it is credible that a further improved effect would be achieved if these components are combined with the ultrafiltered whey protein hydrolysate of the invention.

## Claims

1. A process for preparing an ultrafiltered whey protein hydrolysate, said process comprising:
a) Subjecting whey protein **A** to hydrolysis **1** with one or more proteolytic enzymes thereby obtaining a whey protein hydrolysate **B**, and
b) subjecting the whey protein hydrolysate **B** to membrane ultrafiltration **2** thereby obtaining a permeate **C1** and a retentate **C2,**
wherein
(i) an emulsifier **E** is added to the whey protein **A** prior to or during hydrolysis **1** or wherein an emulsifier and/or a chaotropic agent **E** is added to the whey protein hydrolysate **B** prior to the membrane ultrafiltration **2,**
wherein the ultrafiltered whey protein hydrolysate is in permeate **C1,**
and/or wherein
(ii) after step b)
c) an emulsifier and/or a chaotropic agent **E** is added to retentate **C2** thereby obtaining a retentate **C3,**
d) subjecting the retentate **C3** to membrane ultrafiltration **3** thereby obtaining a permeate **D1** and a retentate **D2,** and
e) adding at least part of the permeate **D1** to the permeate **C1** thereby obtaining permeate **D3,**
wherein the ultrafiltered whey protein hydrolysate is in permeate **D3.**

2. The process according to claim 1, wherein said ultrafiltered whey protein hydrolysate comprises at least one betalactoglobulin peptide comprising an amino acid sequence according to any one of SEQ ID No. 2 - 70, preferably an amino acid sequence according to any one of SEQ ID No. 31-70.

3. The process according to claim 1 or 2, wherein the ultrafiltered whey protein hydrolysate comprises less than 100mg, preferably less than 50mg, more preferably less than 10mg of peptides with a size above 5 kDa per gram protein; and/or wherein the ultrafiltered whey protein hydrolysate comprises less than 30 mg per gram protein, preferably less than 10 mg per gram protein, most preferably less than 5 mg per gram protein of peptides with a size above 10 kDa.

4. The process according to any one of the preceding claims, wherein the ultrafiltered whey protein hydrolysate comprises at least 300 mg of peptides with a size of 1 kDa or below per gram protein, even more preferably at least 500 mg per gram protein; and/or wherein the ultrafiltered whey protein hydrolysate comprises at least 900 mg per gram protein of peptides with a size of 5 kDa or below, even more preferably at least 950 mg per gram protein, most preferably at least 990 mg per gram protein.

5. The process according to any one of the preceding claims wherein the ultrafiltered whey protein hydrolysate has a degree of hydrolysis between 5 and 50 %, preferably between 10 and 30 %, more preferably between 15 and 25 %.

6. The process according to any one of the preceding claims wherein the membrane used in membrane ultrafiltration **2** and/or membrane ultrafiltration **3** has a pore size with a cut-off in the range of 5 - 15 kDa, preferably 5-11 kDa, more preferably 9-11 kDa.

7. The process according to any one of the preceding claims, wherein an emulsifier **E** is added, wherein the emulsifier preferably is selected from the group consisting of citric acid esters of mono and diglycerides (Citrem), diacetyl tartaric acid esters of mono- and diglycerides (Datem), monoacylglycerols (MAG), diacylglycerols (DAG), sucrose esters (SE, E473) and soy lecithin, and combinations thereof, more preferably is or comprises Citrem.

8. The process according to any one of the preceding claims, wherein the emulsifier E is added to the whey protein prior to or during hydrolysis, preferably prior to hydrolysis; and preferably in a concentration of at least 50 mg per gram protein to which it is added and/or in a concentration of at least 1 g per liter.

9. The process according to any one of the preceding claims, wherein a chaotropic agent is added, preferably in a concentration of at least 1 gram per 100 ml and/or at least 25 mg per gram protein to which it is added; and wherein the chaotropic agent preferably is or comprises urea.

10. The process according to claim 10, wherein the chaotropic agent E is added to the whey protein hydrolysate B prior to membrane ultrafiltration.

11. An ultrafiltered whey protein hydrolysate obtainable by the process according to any one of the preceding claims, preferably comprising:
- less than 6 microgram allergenic beta-lactoglobulin per gram protein; and/or less than 3%, preferably less than 1% of peptides with a size of above 5kDa based on total protein, and
- at least one betalactoglobulin peptide comprising an amino acid sequence according to any one of SEQ ID No. 2 - 30, preferably according to any one of SEQ ID No. 31 - 57, more preferably according to any one of SEQ ID No. 58-70; and
- preferably at least 50 mg chaotropic agent per gram protein and/or at least 5 mg emulsifier per gram protein.

12. An ultrafiltered whey protein hydrolysate, wherein the ultrafiltered whey protein hydrolysate comprises peptides with the following size distribution:
60 - 90 % with a size < 1 kDa,
10 - 40 % of peptides with a size of 1 to < 5 kDa,
0 - 0.5 % of peptides with a size of 5 to <10 kDa, and
0 - 0.2 % of peptides with a size > 10 kDa,
based on the total protein of the hydrolysate, and
wherein the ultrafiltered whey protein hydrolysate comprises:
∘ less than 6 microgram allergenic beta-lactoglobulin per gram protein; and/or less than 3%, preferably less than 1% of peptides with a size of above 5kDa based on total protein, and
∘ at least one, preferably at least two, more preferably at least three of betalactoglobulin peptide(s) comprising an amino acid sequence according to any one of SEQ ID No. 66 -70,
wherein the ultrafiltered whey protein hydrolysate is preferably obtainable by the process according to any one of claims 1-10.

13. A whey protein hydrolysate composition comprising ultrafiltered whey protein hydrolysate, in a concentration of at least 0.8 gram of ultrafiltered whey protein hydrolysate per gram protein, wherein the ultrafiltered whey protein hydrolysate comprises:
- less than 6 microgram allergenic beta-lactoglobulin per gram protein; and/or less than 3%, preferably less than 1% of peptides with a size of above 5kDa based on total protein, and
- at least one betalactoglobulin peptide comprising an amino acid sequence according to any one of SEQ ID No. 2 - 70, preferably according to any one of SEQ ID No. 31-70; and
- preferably at least 50 mg urea per gram protein and/or at least 5 mg emulsifier per gram protein.

14. The ultrafiltered whey protein hydrolysate according to any one of claims 11 - 12 or the whey protein hydrolysate composition according to claim 13 wherein the at least one betalactoglobulin peptide comprising an amino acid sequence according to any one of SEQ ID No. 2-70, preferably according to any one of SEQ ID No. 31-70, more preferably according to any one of SEQ ID No. 31 - 57, even more preferably according to any one of SEQ ID No. 58-70, most preferably according to any one of SEQ ID No. 66-70 is present in amount of at least 10 microgram preferably 50 microgram per gram protein.

15. The ultrafiltered whey protein hydrolysate according to any one of claims 11 -12 or the whey protein hydrolysate composition according to claim 13 wherein the ultrafiltered whey protein hydrolysate comprises peptides with the following size distribution:
60 - 90 % with a size < 1 kDa,
10 - 40 % of peptides with a size of 1 to < 5 kDa,
0 - 0.5 % of peptides with a size of 5 to <10 kDa, and
0 - 0.2 % of peptides with a size > 10 kDa,
based on the total protein of the hydrolysate.

16. A nutritional composition comprising the ultrafiltered whey protein hydrolysate according to any of claims 11 - 12 or the whey protein hydrolysate composition according to claim 13, wherein said composition is an infant formula, a follow-on formula or a young child formula, preferably an infant formula or a follow-on formula, wherein the nutritional composition preferably further comprising one or more non-digestible oligosaccharides and/or one or more lactic acid producing bacteria.

17. A nutritional composition according to claim 16 for use in
(i) providing nutrition to; and/or
(ii) treating/preventing allergy in; and/or
(iii) inducing oral immune tolerance against an allergen, preferably a food allergen in subjects suffering from allergy, preferably suffering from food allergy, preferably bovine, ovine and/or caprine milk protein, preferably allergy allergy selected from the group consisting of cow's milk protein allergy, goat's milk protein allergy and sheep's milk protein allergy,
wherein the subjects are preferably children up to 6 years of age, more preferably infants between 0 and 12 months of age.

18. The nutritional composition for use according to claim 17, wherein the allergy is a food allergy, preferably bovine, ovine and/or caprine milk protein allergy, preferably allergy selected from the group consisting of cow's milk protein allergy, goat's milk protein allergy and sheep's milk allergy and/or the allergen is selected from the group consisting of cow's milk protein, goat's milk protein and sheep's milk protein, preferably wherein the allergy is cow's milk protein allergy and/or the allergen is cow's milk protein, and wherein the milk protein is preferably betalactoglobulin.

## Patentansprüche

1. Verfahren zum Herstellen eines ultrafiltrierten Molkenproteinhydrolysats, wobei das Verfahren Folgendes umfasst:
a) Unterziehen des Molkenproteins **A** einer Hydrolyse **1** mit einem oder mehreren proteolytischen Enzymen, wodurch ein Molkenproteinhydrolysat **B** erhalten wird, und
b) Unterziehen des Molkenproteinhydrolysats **B** einer Membranultrafiltration **2,** wodurch ein Permeat **C1** und ein Retentat **C2** erhalten werden,
wobei
(i) dem Molkenprotein **A** vor oder während der Hydrolyse **1** ein Emulgator **E** zugesetzt wird oder wobei dem Molkenproteinhydrolysat **B** vor der Membranultrafiltration **2** ein Emulgator und/oder ein chaotropes Mittel **E** zugesetzt wird,
wobei sich das ultrafiltrierte Molkenproteinhydrolysat in dem Permeat **C1** befindet,
und/oder wobei
(ii) nach Schritt b)
c) dem Retentat **C2** ein Emulgator und/oder ein chaotropes Mittel **E** zugesetzt wird, wodurch ein Retentat **C3** erhalten wird,
d) das Retentat **C3** einer Membranultrafiltration **3** unterzogen wird, wodurch ein Permeat **D1** und ein Retentat **D2** erhalten werden, und
e) mindestens ein Teil des Permeats **D1** dem Permeat **C1** zugegeben wird, wodurch das Permeat **D3** erhalten wird,
wobei sich das ultrafiltrierte Molkenproteinhydrolysat in dem Permeat **D3** befindet.

2. Verfahren nach Anspruch 1, wobei das besagte ultrafiltrierte Molkenproteinhydrolysat mindestens ein Betalactoglobulinpeptid umfasst, das eine Aminosäuresequenz gemäß einer der SEQ ID Nr. 2 - 70 umfasst, vorzugsweise eine Aminosäuresequenz gemäß einer der SEQ ID Nr. 31-70.

3. Verfahren nach Anspruch 1 oder 2, wobei das ultrafiltrierte Molkenproteinhydrolysat weniger als 100 mg, vorzugsweise weniger als 50 mg, besonders bevorzugt weniger als 10 mg Peptide mit einer Größe über 5 kDa pro Gramm Protein umfasst; und/oder wobei das ultrafiltrierte Molkenproteinhydrolysat weniger als 30 mg pro Gramm Protein, vorzugsweise weniger als 10 mg pro Gramm Protein, am bevorzugtesten weniger als 5 mg pro Gramm Protein Peptide mit einer Größe über 10 kDa umfasst.

4. Verfahren nach einem der vorhergehenden Ansprüche, wobei das ultrafiltrierte Molkenproteinhydrolysat mindestens 300 mg Peptide mit einer Größe von 1 kDa oder weniger pro Gramm Protein, noch bevorzugter mindestens 500 mg pro Gramm Protein umfasst; und/oder wobei das ultrafiltrierte Molkenproteinhydrolysat mindestens 900 mg pro Gramm Protein Peptide mit einer Größe von 5 kDa oder weniger, noch bevorzugter mindestens 950 mg pro Gramm Protein, am bevorzugtesten mindestens 990 mg pro Gramm Protein umfasst.

5. Verfahren nach einem der vorhergehenden Ansprüche, wobei das ultrafiltrierte Molkenproteinhydrolysat einen Hydrolysegrad zwischen 5 und 50 %, vorzugsweise zwischen 10 und 30 %, besonders bevorzugt zwischen 15 und 25 % aufweist.

6. Verfahren nach einem der vorhergehenden Ansprüche, wobei die in der Membranultrafiltration 2 und/oder der Membranultrafiltration 3 verwendete Membran eine Porengröße mit einem Cut-off im Bereich von 5 - 15 kDa, vorzugsweise 5 - 11 kDa, besonders bevorzugt 9 - 11 kDa aufweist.

7. Verfahren nach einem der vorhergehenden Ansprüche, wobei ein Emulgator **E** zugesetzt wird, wobei der Emulgator vorzugsweise aus der Gruppe ausgewählt ist, die aus Folgendem besteht: Zitronensäureestern von Mono- und Diglyceriden (Citrem), Diazetylweinsäureestern von Mono- und Diglyceriden (Datem), Monoacylglycerinen (MAG), Diacylglycerinen (DAG), Saccharoseestern (SE, E473) und Sojalecithin und Kombinationen davon, besonders bevorzugt Citrem ist oder umfasst.

8. Verfahren nach einem der vorhergehenden Ansprüche, wobei der Emulgator **E** dem Molkenprotein vor oder während der Hydrolyse, vorzugsweise vor der Hydrolyse zugesetzt wird; und vorzugsweise in einer Konzentration von mindestens 50 mg pro Gramm Protein, dem er zugesetzt wird, und/oder in einer Konzentration von mindestens 1 g pro Liter.

9. Verfahren nach einem der vorhergehenden Ansprüche, wobei ein chaotropes Mittel zugesetzt wird, vorzugsweise in einer Konzentration von mindestens 1 Gramm pro 100 ml und/oder mindestens 25 mg pro Gramm Protein, dem es zugesetzt wird; und wobei das chaotrope Mittel vorzugsweise Harnstoff ist oder umfasst.

10. Verfahren nach Anspruch 10, wobei das chaotrope Mittel **E** dem Molkenproteinhydrolysat **B** vor der Membranultrafiltration zugesetzt wird.

11. Ultrafiltriertes Molkenproteinhydrolysat, das durch das Verfahren nach einem der vorhergehenden Ansprüche erhältlich ist, das vorzugsweise Folgendes umfasst:
- weniger als 6 Mikrogramm allergenes Beta-Lactoglobulin pro Gramm Protein; und/oder weniger als 3 %, vorzugsweise weniger als 1 % Peptide mit einer Größe von über 5 kDa, bezogen auf das Gesamtprotein, und
- mindestens ein Betalactoglobulinpeptid, das eine Aminosäuresequenz gemäß einer der SEQ ID Nr. 2 - 30, vorzugsweise gemäß einer der SEQ ID Nr. 31 - 57, besonders bevorzugt gemäß einer der SEQ ID Nr. 58-70 umfasst; und
- vorzugsweise mindestens 50 mg chaotropes Mittel pro Gramm Protein und/oder mindestens 5 mg Emulgator pro Gramm Protein.

12. Ultrafiltriertes Molkenproteinhydrolysat, wobei das ultrafiltrierte Molkenproteinhydrolysat Peptide mit der folgenden Größenverteilung umfasst:
60 - 90 % mit einer Größe < 1 kDa,
10 - 40 % Peptide mit einer Größe von 1 bis < 5 kDa,
0 - 0,5 % Peptide mit einer Größe von 5 bis <10 kDa, und
0 - 0,2 % Peptide mit einer Größe > 10 kDa, bezogen auf das Gesamtprotein des Hydrolysats, und
wobei das ultrafiltrierte Molkenproteinhydrolysat Folgendes umfasst:
o weniger als 6 Mikrogramm allergenes Beta-Lactoglobulin pro Gramm Protein; und/oder weniger als 3 %, vorzugsweise weniger als 1 % Peptide mit einer Größe von über 5kDa, bezogen auf das Gesamtprotein, und
o mindestens ein, vorzugsweise mindestens zwei, besonders bevorzugt mindestens drei Betalactoglobulinpeptid(e), die eine Aminosäuresequenz gemäß einer der SEQ ID Nr. 66 -70 umfassen,
wobei das ultrafiltrierte Molkenproteinhydrolysat vorzugsweise durch das Verfahren nach einem der Ansprüche 1 - 10 erhältlich ist.

13. Molkenproteinhydrolysat-Zusammensetzung, die ultrafiltriertes Molkenproteinhydrolysat in einer Konzentration von mindestens 0,8 Gramm ultrafiltriertem Molkenproteinhydrolysat pro Gramm Protein umfasst, wobei das ultrafiltrierte Molkenproteinhydrolysat Folgendes umfasst:
- weniger als 6 Mikrogramm allergenes Beta-Lactoglobulin pro Gramm Protein; und/oder weniger als 3 %, vorzugsweise weniger als 1 % Peptide mit einer Größe von über 5 kDa, bezogen auf das Gesamtprotein, und
- mindestens ein Betalactoglobulinpeptid, das eine Aminosäuresequenz gemäß einer der SEQ ID Nr. 2 - 70, vorzugsweise gemäß einer der SEQ ID Nr. 31-70 umfasst; und
- vorzugsweise mindestens 50 mg Harnstoff pro Gramm Protein und/oder mindestens 5 mg Emulgator pro Gramm Protein.

14. Ultrafiltriertes Molkenproteinhydrolysat nach einem der Ansprüche 11 - 12 oder Molkenproteinhydrolysat-Zusammensetzung nach Anspruch 13, wobei das mindestens eine Betalactoglobulinpeptid, das eine Aminosäuresequenz gemäß einer der SEQ ID Nr. 2-70, vorzugsweise gemäß einer der SEQ ID Nr. 31-70, besonders bevorzugt gemäß einer der SEQ ID Nr. 31 - 57, noch bevorzugter gemäß einer der SEQ ID Nr. 58-70, am bevorzugtesten gemäß einer der SEQ ID Nr. 66-70 umfasst, in einer Menge von mindestens 10 Mikrogramm, vorzugsweise 50 Mikrogramm pro Gramm Protein vorhanden ist.

15. Ultrafiltriertes Molkenproteinhydrolysat nach einem der Ansprüche 11 - 12 oder Molkenproteinhydrolysat-Zusammensetzung nach Anspruch 13, wobei das ultrafiltrierte Molkenproteinhydrolysat Peptide mit der folgenden Größenverteilung umfasst:
60 - 90 % mit einer Größe < 1 kDa,
10 - 40 % Peptide mit einer Größe von 1 bis < 5 kDa,
0 - 0,5 % Peptide mit einer Größe von 5 bis <10 kDa, und
0 - 0,2 % Peptide mit einer Größe > 10 kDa,
bezogen auf das Gesamtprotein des Hydrolysats.

16. Ernährungszusammensetzung, die das ultrafiltrierte Molkenproteinhydrolysat nach einem der Ansprüche 11 - 12 oder die Molkenproteinhydrolysat-Zusammensetzung nach Anspruch 13 umfasst, wobei die besagte Zusammensetzung eine Säuglingsnahrung, eine Folgenahrung oder eine Kleinkindnahrung ist, vorzugsweise eine Säuglingsnahrung oder eine Folgenahrung, wobei die Ernährungszusammensetzung vorzugsweise ferner ein oder mehrere unverdauliche Oligosaccharide und/oder eine oder mehrere Milchsäure produzierende Bakterien umfasst.

17. Ernährungszusammensetzung nach Anspruch 16 zur Verwendung bei dem
(i) Bereitstellen von Nahrung; und/oder
(ii) Behandeln/Vorbeugen von Allergien; und/oder
(iii) Induzieren einer oralen Immuntoleranz gegen ein Allergen, vorzugsweise ein Nahrungsmittelallergen, bei Patienten, die an einer Allergie leiden, die vorzugsweise an einer Nahrungsmittelallergie leiden, vorzugsweise einer Kuh-, Schafs- und/oder Ziegenmilchproteinallergie, vorzugsweise an einer Allergie, die aus der Gruppe ausgewählt ist, die aus Kuhmilchproteinallergie, Ziegenmilchproteinallergie und Schafsmilchproteinallergie besteht,
wobei die Patienten vorzugsweise Kinder im Alter von bis zu 6 Jahren sind, besonders bevorzugt Säuglinge im Alter zwischen 0 und 12 Monaten.

18. Ernährungszusammensetzung zur Verwendung nach Anspruch 17, wobei die Allergie eine Nahrungsmittelallergie ist, vorzugsweise Kuh-, Schafs- und/oder Ziegenmilchproteinallergie, vorzugsweise eine Allergie, die aus der Gruppe ausgewählt ist, die aus Kuhmilchproteinallergie, Ziegenmilchproteinallergie und Schafsmilchproteinallergie besteht, und/oder das Allergen aus der Gruppe ausgewählt ist, die aus Kuhmilchprotein, Ziegenmilchprotein und Schafsmilchprotein besteht, vorzugsweise wobei die Allergie eine Kuhmilchproteinallergie ist und/oder das Allergen Kuhmilchprotein ist, und wobei das Milchprotein vorzugsweise Betalactoglobulin ist.

## Revendications

1. Procédé de préparation d'un hydrolysat de protéine de lactosérum ultrafiltré, ledit procédé comprenant :
a) soumettre la protéine de lactosérum A à une hydrolyse 1 avec une ou plusieurs enzymes protéolytiques obtenant ainsi un hydrolysat de protéine de lactosérum B, et
b) soumettre l'hydrolysat de protéine de lactosérum B à une ultrafiltration membranaire 2 obtenant ainsi un perméat C1 et un rétentat C2,
où
(i) un émulsifiant E est ajouté à la protéine de lactosérum A avant ou pendant l'hydrolyse 1 ou où un émulsifiant et/ou un agent chaotropique E est ajouté à l'hydrolysat de protéine de lactosérum B avant l'ultrafiltration membranaire 2,
où l'hydrolysat de protéine de lactosérum ultrafiltré est dans le perméat C1,
et/ou où
(ii) après l'étape b)
c) un émulsifiant et/ou un agent chaotropique E est ajouté au rétentat C2 obtenant ainsi un rétentat C3,
d) soumettre le rétentat C3 à une ultrafiltration membranaire 3 obtenant ainsi un perméat D1 et un rétentat D2, et
e) ajouter au moins une partie du perméat D1 au perméat C1 obtenant ainsi le perméat D3,
où l'hydrolysat de protéine de lactosérum ultrafiltré est dans le perméat D3.

2. Procédé selon la revendication 1, où ledit hydrolysat de protéine de lactosérum ultrafiltré comprend au moins un peptide de bêta-lactoglobuline comprenant une séquence d'acides aminés selon l'une quelconque des SEQ ID No. 2 - 70, de préférence une séquence d'acides aminés selon l'une quelconque des SEQ ID No. 31 - 70.

3. Procédé selon la revendication 1 ou 2, où l'hydrolysat de protéine de lactosérum ultrafiltré comprend moins de 100 mg, de préférence moins de 50 mg, plus préférablement moins de 10 mg de peptides avec une taille supérieure à 5 kDa par gramme de protéine ; et/ou où l'hydrolysat de protéine de lactosérum ultrafiltré comprend moins de 30 mg par gramme de protéine, de préférence moins de 10 mg par gramme de protéine, plus préférablement moins de 5 mg par gramme de protéine de peptides avec une taille supérieure à 10 kDa.

4. Procédé selon l'une quelconque des revendications précédentes, où l'hydrolysat de protéine de lactosérum ultrafiltré comprend au moins 300 mg de peptides avec une taille de 1 kDa ou inférieure par gramme de protéine, encore plus préférablement au moins 500 mg par gramme de protéine ; et/ou où l'hydrolysat de protéine de lactosérum ultrafiltré comprend au moins 900 mg par gramme de protéine de peptides d'une taille de 5 kDa ou inférieure, encore plus préférablement au moins 950 mg par gramme de protéine, plus préférentiellement au moins 990 mg par gramme de protéine.

5. Procédé selon l'une quelconque des revendications précédentes, où l'hydrolysat de protéine de lactosérum ultrafiltré a un degré d'hydrolyse entre 5 et 50 %, de préférence entre 10 et 30 %, plus préférablement entre 15 et 25 %.

6. Procédé selon l'une quelconque des revendications précédentes où la membrane utilisée dans l'ultrafiltration membranaire 2 et/ou l'ultrafiltration membranaire 3 a une taille de pore avec un seuil de coupure dans la gamme de 5 à 15 kDa, de préférence de 5 à 11 kDa, plus préférablement de 9 à 11 kDa.

7. Procédé selon l'une quelconque des revendications précédentes, où un émulsifiant E est ajouté, où l'émulsifiant est de préférence sélectionné dans le groupe constitué d'esters d'acide citrique de mono et diglycérides (Citrem), d'esters d'acide diacétyltartrique de mono- et diglycérides (Datem), de monoacylglycérols (MAG), de diacylglycérols (DAG), d'esters de saccharose (SE, E473) et de lécithine de soja, et des combinaisons de ceux-ci, plus préférablement est ou comprend du Citrem.

8. Procédé selon l'une quelconque des revendications précédentes, où l'émulsifiant E est ajouté à la protéine de lactosérum avant ou pendant l'hydrolyse, de préférence avant l'hydrolyse ; et de préférence à une concentration d'au moins 50 mg par gramme de protéine à laquelle il est ajouté et/ou à une concentration d'au moins 1 g par litre.

9. Procédé selon l'une quelconque des revendications précédentes, où un agent chaotropique est ajouté, de préférence à une concentration d'au moins 1 gramme par 100 ml et/ou d'au moins 25 mg par gramme de protéine à laquelle il est ajouté ; et où l'agent chaotropique de préférence est ou comprend de l'urée.

10. Procédé selon la revendication 10, où l'agent chaotropique E est ajouté à l'hydrolysat de protéine de lactosérum B avant l'ultrafiltration membranaire.

11. Hydrolysat de protéine de lactosérum ultrafiltré obtenu par le procédé selon l'une quelconque des revendications précédentes, comprenant de préférence :
- moins de 6 microgrammes de bêta-lactoglobuline allergène par gramme de protéine ; et/ou moins de 3 %, de préférence moins de 1 % de peptides avec une taille supérieure à 5kDa sur la base de protéine totale, et
- au moins un peptide de bêta-lactoglobuline comprenant une séquence d'acides aminés selon l'une quelconque des SEQ ID No. 2 - 30, de préférence selon l'une quelconque des SEQ ID No. 31 - 57, plus préférablement selon l'une quelconque des SEQ ID No. 58 - 70 ; et
- de préférence au moins 50 mg d'agent chaotropique par gramme de protéine et/ou au moins 5 mg d'émulsifiant par gramme de protéine.

12. Hydrolysat de protéine de lactosérum ultrafiltré, où l'hydrolysat de protéine de lactosérum ultrafiltré comprend des peptides avec la distribution de taille suivante :
60 à 90 % avec une taille < 1 kDa,
10 à 40 % de peptides avec une taille de 1 à < 5 kDa,
0 à 0,5 % de peptides avec une taille de 5 à < 10 kDa, et
0 à 0,2 % de peptides avec une taille > 10 kDa,
sur la base de la protéine totale de l'hydrolysat, et
où l'hydrolysat de protéine de lactosérum ultrafiltré comprend :
∘ moins de 6 microgrammes de bêta-lactoglobuline allergène par gramme de protéine ; et/ou moins de 3 %, de préférence moins de 1 % de peptides avec une taille supérieure à 5 kDa sur la base de protéine totale, et
∘ au moins un, de préférence au moins deux, plus préférablement au moins trois peptide(s) de bêta-lactoglobuline comprenant une séquence d'acides aminés selon l'une quelconque des SEQ ID No. 66 - 70,
où l'hydrolysat de protéine de lactosérum ultrafiltré est de préférence obtenu par le procédé selon l'une quelconque des revendications 1 à 10.

13. Composition d'hydrolysat de protéine de lactosérum comprenant un hydrolysat de protéine de lactosérum ultrafiltré, dans une concentration d'au moins 0,8 gramme d'hydrolysat de protéine de lactosérum ultrafiltré par gramme de protéine, où l'hydrolysat de protéine de lactosérum ultrafiltré comprend :
- moins de 6 microgrammes de bêta-lactoglobuline allergène par gramme de protéine ; et/ou moins de 3 %, de préférence moins de 1 % de peptides avec une taille supérieure à 5kDa sur la base de protéine totale, et
- au moins un peptide de bêta-lactoglobuline comprenant une séquence d'acides aminés selon l'une quelconque des SEQ ID No. 31 - 70, de préférence selon l'une quelconque des SEQ ID No. 31 - 70 ; et
- de préférence au moins 50 mg d'urée par gramme de protéine et/ou au moins 5 mg d'émulsifiant par gramme de protéine.

14. Hydrolysat de protéine de lactosérum ultrafiltré selon l'une quelconque des revendications 11 à 12 ou la composition d'hydrolysat de protéine de lactosérum selon la revendication 13, où l'au moins un peptide de bêta-lactoglobuline comprenant une séquence d'acides aminés selon l'une quelconque des SEQ ID No. 2 - 70, de préférence selon l'une quelconque des SEQ ID No. 31 - 70, plus préférablement selon l'une quelconque des SEQ ID No. 31 - 57, encore plus préférablement selon l'une quelconque des SEQ ID No. 58 - 70, plus préférablement selon l'une quelconque des SEQ ID No. 66 - 70 est présent en quantité d'au moins 10 microgrammes de préférence 50 microgrammes par gramme de protéine.

15. Hydrolysat de protéine de lactosérum ultrafiltré selon l'une quelconque des revendications 11 à 12 ou la composition d'hydrolysat de protéine de lactosérum selon la revendication 13 où l'hydrolysat de protéine de lactosérum ultrafiltré comprend des peptides avec la distribution de taille suivante :
60 à 90 % avec une taille < 1 kDa,
10 à 40 % de peptides avec une taille de 1 à < 5 kDa,
0 à 0,5 % de peptides avec une taille de 5 à < 10 kDa, et
0 à 0,2 % de peptides avec une taille > 10 kDa,
sur la base de la protéine totale de l'hydrolysat.

16. Composition nutritionnelle comprenant l'hydrolysat de protéine de lactosérum ultrafiltré selon l'une quelconque des revendications 11 à 12 ou la composition d'hydrolysat de protéine de lactosérum selon la revendication 13, où ladite composition est une préparation pour nourrisson, une préparation de suite ou une préparation pour jeune enfant, de préférence une préparation pour nourrisson ou une préparation de suite, où la composition nutritionnelle comprend en outre de préférence un ou plusieurs oligosaccharides non digestibles et/ou une ou plusieurs bactéries produisant de l'acide lactique.

17. Composition nutritionnelle selon la revendication 16 pour utilisation en
(i) fournir une alimentation à ; et/ou
(ii) traiter/prévenir des allergies en ; et/ou
(iii) induire une tolérance immunitaire orale contre un allergène, de préférence un allergène alimentaire chez des sujets souffrant d'allergie, de préférence souffrant d'allergie alimentaire, de préférence aux protéines de lait bovin, ovin et/ou caprin, de préférence une allergie sélectionnée dans le groupe constitué d'allergie aux protéines de lait de vache, d'allergie aux protéines de lait de chèvre et d'allergie aux protéines de lait de brebis,
où les sujets sont de préférence des enfants jusqu'à l'âge de 6 ans, plus préférablement des nourrissons entre 0 et 12 mois d'âge.

18. Composition nutritionnelle pour utilisation selon la revendication 17, où l'allergie est une allergie alimentaire, de préférence une allergie aux protéines de lait bovin, ovin et/ou caprin, de préférence une allergie sélectionnée dans le groupe constitué d'allergie aux protéines de lait de vache, d'allergie aux protéines de lait de chèvre et d'allergie aux protéines de lait de brebis, et/ou l'allergène est sélectionné dans le groupe constitué de protéines de lait de vache, de protéines de lait de chèvre et de protéines de lait de brebis, de préférence où l'allergie est une allergie aux protéines de lait de vache et/ou l'allergène est une protéine de lait de vache, et où la protéine de lait est de préférence une bêta-lactoglobuline.
